(19) Europäisches Patentamt
European Patent Office
Office européen des brevets

(11) EP 3 148 646 B2

(12) # NEW EUROPEAN PATENT SPECIFICATION

After opposition procedure

(45) Date of publication and mention of the opposition decision:
**03.08.2022 Bulletin 2022/31**

(45) Mention of the grant of the patent:
**21.08.2019 Bulletin 2019/34**

(21) Application number: **15731700.9**

(22) Date of filing: **27.05.2015**

(51) International Patent Classification (IPC):
***A61Q 19/00*** *(2006.01)* ***A61K 8/04*** *(2006.01)*
***A61K 8/25*** *(2006.01)* ***A61K 8/81*** *(2006.01)*
***A61K 8/89*** *(2006.01)* ***A61K 8/891*** *(2006.01)*

(52) Cooperative Patent Classification (CPC):
**A61K 8/042; A61K 8/25; A61K 8/8141; A61K 8/8152; A61K 8/891; A61Q 19/00; A61Q 19/001**

(86) International application number:
**PCT/IB2015/053953**

(87) International publication number:
**WO 2015/181733 (03.12.2015 Gazette 2015/48)**

(54) **COSMETIC COMPOSITION FOR MAKE UP AND FOR TAKING CARE OF KERATIN MATERIALS**

KOSMETISCHE ZUSAMMENSETZUNG FÜR MAKE UP UND ZUR PFLEGE VON KERATINMATERIALIEN

COMPOSITIONS COSMÉTIQUES POUR MAQUILLAGE ET POUR SOIGNER LES MATÉRIAUX DE KÉRATINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2014 FR 1454855**

(43) Date of publication of application:
**05.04.2017 Bulletin 2017/14**

(73) Proprietor: **L'Oréal**
**75008 Paris (FR)**

(72) Inventors:
- **BCHIR, Olfa**
  **F-75013 Paris (FR)**
- **CHEVALIER, Gaëtan**
  **F-45760 Boigny Sur Bionne (FR)**
- **VALVERDE, Elodie**
  **26400 GIGORS-ET-LOZERON (FR)**

(74) Representative: **Nony**
**11 rue Saint-Georges**
**75009 Paris (FR)**

(56) References cited:
**EP-A1- 1 634 576**
**EP-A1- 1 671 673**
**EP-A1- 2 492 333**
**EP-A2- 0 388 582**
**EP-A2- 1 360 955**
**WO-A1-99/22696**
**WO-A1-2005/070384**
**WO-A1-2009/007307**
**WO-A2-2013/093869**
**US-A1- 2005 196 364**
**US-A1- 2008 299 156**
**US-A1- 2008 299 157**

- **DATABASE WPI [Online] THOMSON SCIENTIFIC, LONDON, GB; 14 September 1989 (1989-09-14), (KOBA-N) KOBAYASHI KASE KENK, (KOBA-N) KOBAYASHI KOSE KK: "OIL UP COSMETIC MATERIAL CONSIST DISPERSE SYSTEM SILICONE GEL COMPOSITION POLYSILOXANE POWDER BASE AGENT", XP002735135, Database accession no. 1989-312786 & JP H01 230511 A (KOBAYASHI KOOC KENKYUSHO KK) 14 September 1989 (1989-09-14)**

## Description

The present invention is directed towards proposing for the field of caring for and/or making up keratin materials, especially the skin and/or the lips, and in particular the skin, a novel galenical form that is most particularly advantageous with regard to its technical performance and the sensations it affords the user during its application thereto, in particular to the skin.

The term *"keratin materials"* especially means the skin, the lips and/or the eyelashes, in particular the skin and/or the lips, and preferably the skin.

Cosmetic compositions are commonly employed for hiding and/or unifying skin relief imperfections such as pores, wrinkles and/or fine lines and/or scars. In this regard, many solid or fluid, anhydrous or non-anhydrous formulations have been developed to date.

When these compositions are more particularly intended for fading out the visibility of the skin relief, the formulator uses therein diffusing fillers or soft-focus fillers. However, the corresponding compositions that are currently available do not prove to be entirely satisfactory, especially in terms of soft-focus performance.

There is thus still a need for mattifying and/or smoothing cosmetic compositions that can mask skin imperfections, which have good cosmetic properties, in particular which are soft, fresh and light on application.

The present invention is specifically directed towards meeting this need.

Thus, according to one of its aspects, the present invention relates to a cosmetic composition, different from an emulsion, for making up and/or caring for keratin materials, in particular the skin and/or the lips, comprising:

- at least one aqueous phase gelled with at least one synthetic polymeric hydrophilic gelling agent chosen from crosslinked and/or neutralized 2-acrylamido-2-methylpropanesulfonic acid copolymers; and
- at least one oily phase gelled with at least one lipophilic gelling agent chosen from organopolysiloxane elastomers;

said phases forming therein a macroscopically homogeneous mixture;
said composition also comprising at least one soft-focus filler chosen from acrylic copolymer powders, polymethyl methacrylate powders, talc/$TiO_2$/alumina/silica composite powders, silica/ $TiO_2$ composite powders, hollow hemispherical silicone particles, crosslinked elastomeric organopolysiloxane powders, crosslinked elastomeric organopolysiloxane powders coated with silicone resin, starch powders, polyamide powders, spherical cellulose beads, and mixtures thereof.

In the context of the present invention, the term "soft-focus effect" denotes a hazy effect which hides the skin's microreliefs. This effect makes it possible especially to attenuate via an optical effect skin defects such as marks, wrinkles or fine lines.

These soft-focus fillers may also be referred to as fillers with a soft-focus effect or haze-effect fillers.

Contrary to all expectation, and as emerges from the examples given below, the inventors have found that the formulation of a soft-focus filler in a gel-gel architecture as defined above makes it possible to boost this soft-focus effect. Furthermore, the sensation of discomfort liable to be generated by the presence of this type of filler is no longer experienced on application.

"Gel-gel" compositions have already been proposed in the cosmetics field. Formulations of this type combine a gelled aqueous phase with a gelled oily phase. Thus, gel/gel formulations are described in Almeida et al., Pharmaceutical Development and Technology, 2008, 13:487, tables 1 and 2, page 488; WO 99/65455; PI 04057589; WO 99/62497; JP 2005-112834; WO 2013/093869 and WO 2008/081175. However, to the inventors' knowledge, this type of composition does not currently make it possible to dissimulate and smooth out relief imperfections without thereby impairing the other expected cosmetic performance qualities. WO 99/22696 relates to anhydrous makeup compositions comprising a silicone gel comprising an organopolysiloxane elastomer dispersed in a silicone-compatible vehicle, the compositions producing a matte or non-shiny appearance when applied to the skin. The compositions of WO 99/22696 are not in a form of a gel/gel formulation.

According to one embodiment variant, a composition according to the invention may comprise from 0.2% to 40% by weight, especially from 0.5% to 37% by weight, in particular from 0.75% to 35% by weight and preferably from 1% to 30% by weight of soft-focus filler(s) relative to the total weight of said composition.

According to an advantageous embodiment variant, said soft-focus filler(s) are totally or partly, and preferably solely, present in the gelled aqueous phase or are totally or partly, and preferably solely, present in the gelled oily phase.

As stated above, the inventors have found that the choice of a particular hydrophilic gelling agent for texturing the aqueous phase of a composition of gel/gel type and the use of soft-focus fillers in a composition according to the invention makes it possible to duplicate the effects of these fillers, as illustrated in the examples.

Thus, a composition according to the invention shows very good properties in terms of hiding relief imperfections and of smoothing out the skin, while at the same time affording the user a sensation of freshness and lightness. Finally, the composition proves to be easy to apply to the surface of the targeted keratin material.

According to another of its aspects, a subject of the invention is also a process for preparing a cosmetic

composition, different from an emulsion, for making up and/or caring for keratin materials, in particular the skin and/or the lips, comprising at least one step of mixing:

- an aqueous phase gelled with at least one synthetic polymeric hydrophilic gelling agent chosen from crosslinked and/or neutralized 2-acrylamido-2-methylpropanesulfonic acid copolymers; and
- at least one oily phase gelled with at least one lipophilic gelling agent chosen from organopolysiloxane elastomers;

under conditions suitable for obtaining a macroscopically homogeneous mixture;
said composition also comprising at least one soft-focus filler chosen from acrylic copolymer powders, polymethyl methacrylate powders, talc/$TiO_2$/alumina/silica composite powders, silica/$TiO_2$composite powders, hollow hemispherical silicone particles, crosslinked elastomeric organopolysiloxane powders, crosslinked elastomeric organopolysiloxane powders coated with silicone resin, starch powders, polyamide powders, spherical cellulose beads, and mixtures thereof.

According to one embodiment variant, this process may advantageously comprise a step of mixing at least three or even more gelled phases.

For obvious reasons, the number of gelled aqueous phases and of gelled oily phases to be considered for forming a composition according to the invention may range for each of the two types of phase beyond two.

Advantageously, the mixing of the phases may be performed at room temperature.

However, the process of the invention may comprise, if necessary, a step of heating the mixture.

According to a particular embodiment, the representative gelled phases of the same type of architecture are gelled with a different gelling agent.

Multi-phase formulas may thus be developed.

According to another of its aspects, a subject of the invention is also a process, especially a cosmetic process, for making up and/or caring for a keratin material, in particular the skin and/or the lips, comprising at least one step which consists in applying to said keratin material a composition in accordance with the invention.

According to yet another of its aspects, the present invention relates to a cosmetic process for making up and/or caring for a keratin material, in particular the skin and/or the lips, comprising at least the application to said material of a macroscopically homogeneous composition, different from an emulsion, obtained by extemporaneous mixing, before application or at the time of application to said keratin material, of at least one aqueous phase gelled with at least one synthetic polymeric hydrophilic gelling agentchosen from crosslinked and/or neutralized 2-acrylamido-2-methylpropanesulfonic acid copolymers, and of at least one oily phase gelled with at least one lipophilic gelling agent chosen from organopolysiloxane elastomers; and said composition also comprising at least one soft-focus filler chosen from acrylic copolymer powders, polymethyl methacrylate powders, talc/$TiO_2$/alumina/silica composite powders, silica/$TiO_2$ composite powders, hollow hemispherical silicone particles, crosslinked elastomeric organopolysiloxane powders, crosslinked elastomeric organopolysiloxane powders coated with silicone resin, starch powders, polyamide powders, spherical cellulose beads, and mixtures thereof.

## COSMETIC COMPOSITION

To begin with, it is important to note that a composition according to the invention is different from an emulsion.

An emulsion generally consists of an oily liquid phase and an aqueous liquid phase. It is a dispersion of droplets of one of the two liquid phases in the other. The size of the droplets forming the dispersed phase of the emulsion is typically about a micrometre (0.1 to 100 $\mu$m). Furthermore, an emulsion requires the presence of a surfactant or of an emulsifier to ensure its stability over time.

In contrast, a composition according to the invention consists of a macroscopically homogeneous mixture of two immiscible gelled phases. These two phases both have a gel-type texture. This texture is especially reflected visually by a consistent and/or creamy appearance.

The term *"macroscopically homogeneous mixture"* means a mixture in which each of the gelled phases cannot be individualized by the naked eye.

More precisely, in a composition according to the invention, the gelled aqueous phase and the gelled oily phase interpenetrate and thus form a stable, consistent product. This consistency is achieved by mixing interpenetrated macrodomains. These interpenetrated macrodomains are not measurable objects. Thus, by microscope, the composition according to the invention is very different from an emulsion. A composition according to the invention cannot be characterized either as having a *"sense",* i.e. an O/W or W/O sense.

Thus, a composition according to the invention has a consistency of gel type. The stability of the composition is long-lasting without surfactant. Consequently, a cosmetic composition according to the invention does not require any surfactant or silicone emulsifier to ensure its stability over time.

It is known practice from the prior art to observe the intrinsic nature of a mixture of aqueous and oily gels in a gel-type composition, for example, by introducing a dyestuff either into the aqueous gelled phase or into the lipophilic

gelled phase, before the formation of the gel-type composition. During visual inspection, in a gel-type composition, the dyestuff appears uniformly dispersed, even if the dye is present solely in the gelled aqueous phase or in the gelled oily phase. Specifically, if two different dyes of different colours are introduced, respectively, into the oily phase and into the aqueous phase, before formation of the gel-type composition, the two colours may be observed as being uniformly dispersed throughout the gel-type composition. This is different from an emulsion in which, if a dye, which is soluble in water or soluble in oil, is introduced, respectively, into the aqueous and oily phases, before forming the emulsion, the colour of the dye present will only be observed in the outer phase (Remington: The Science and Practice of Pharmacy, 19th Edition (1995), Chapter 21, page 282).

It is also known practice to distinguish a gel-type composition from an emulsion by performing a "drop test". This test consists in demonstrating the bi-continuous nature of a gel-type composition. Specifically, as mentioned previously, the consistency of a composition is obtained by means of the interpenetration of the aqueous and oily gelled domains. Consequently, the bi-continuous nature of a gel-type composition may be demonstrated by means of a simple test with, respectively, hydrophilic and hydrophobic solvents. This test consists in depositing, firstly, one drop of a hydrophilic solvent on a first sample of the test composition, and, secondly, one drop of a hydrophobic solvent on a second sample of the same test composition, and in analysing the behaviour of the two drops of solvents. In the case of an O/W emulsion, the drop of hydrophilic solvent diffuses into the sample and the drop of hydrophobic solvent remains at the surface of the sample. In the case of a W/O emulsion, the drop of hydrophilic solvent remains at the surface of the sample and the drop of hydrophobic solvent diffuses throughout the sample. Finally, in the case of a gel-type composition (bi-continuous system), the hydrophilic and hydrophobic drops diffuse throughout the sample.

In the case of the present invention, the test that will be preferred for distinguishing a gel-type composition from an emulsion is a dilution test. Specifically, in a gel-type composition, the aqueous and oily gelled domains interpenetrate and form a consistent and stable composition, in which the behaviour in water and in oil is different from the behaviour of an emulsion. Consequently, the behaviour during dilution of a gel-type composition (bi-continuous system) may be compared to that of an emulsion.

More specifically, the dilution test consists in placing 40 g of product and 160 g of dilution solvent (water or oil) in a 30 mL plastic beaker. The dilution is performed with controlled stirring to avoid any emulsification. In particular, this is performed using a planetary mixer: Speed Mixer TM DAC400FVZ. The speed of the mixer is set at 1500 rpm for 4 minutes. Finally, observation of the resulting sample is performed using an optical microscope at a magnification of ×100 (× 10× 10). It may be noted that oils such as Parleam® and Xiameter PMX-200 Silicone Fluid 5CS® sold by Dow Corning are suitable as dilution solvent.

In the case of a gel-type composition (bi-continuous system), when it is diluted in oil or in water, a heterogeneous appearance is always observed. When a gel-type composition (bi-continuous system) is diluted in water, pieces of oily gel in suspension are observed, and when a gel-type composition (bi-continuous system) is diluted in oil, pieces of aqueous gel in suspension are observed.

In contrast, during dilution, emulsions have a different behaviour. When an O/W emulsion is diluted in an aqueous solvent, it gradually reduces without having a heterogeneous and lumpy appearance. This same O/W emulsion, on dilution with oil, has a heterogeneous appearance (pieces of O/W emulsion suspended in the oil). When a W/O emulsion is diluted with an aqueous solvent, it has a heterogeneous appearance (pieces of W/O emulsion suspended in the water). This same W/O emulsion, when diluted in oil, gradually reduces without having a heterogeneous and lumpy appearance.

According to the present invention, the aqueous gelled phase and the oily gelled phase forming a composition according to the invention are present therein in a weight ratio ranging from 95/5 to 5/95. More preferentially, the aqueous phase and the oily phase are present in a weight ratio ranging from 30/70 to 80/20.

The ratio between the two gelled phases is adjusted according to the desired cosmetic properties.

Thus, in the case of a makeup composition, in particular for the face, it may be advantageous to favour an aqueous gelled phase/oily gelled phase weight ratio of greater than 1, especially ranging from 60/40 to 90/10, preferably ranging from 60/40 to 80/20, preferentially from 60/40 to 70/30 and even more preferentially to favour an aqueous gelled phase/oily gelled phase weight ratio of 60/40 or 70/30.

These preferred ratios are particularly advantageous for obtaining fresh and light compositions.

Advantageously, a composition according to the invention is thus in the form of a creamy gel with a minimum stress below which it does not flow unless it has been subjected to an external mechanical stress.

As emerges from the text hereinbelow, a composition according to the invention may have a minimum threshold stress of 1.5 Pa and in particular greater than 10 Pa.

It also advantageously has a stiffness modulus G* at least equal to 400 Pa and preferably greater than 1000 Pa.

According to an advantageous embodiment variant, the gelled phases under consideration to form a composition according to the invention have, respectively, a threshold stress of greater than 1.5 Pa and preferably greater than 10 Pa.

Characterization of the threshold stresses is performed by oscillating rheology measurements. Methodology is proposed in the illustrative chapter of the present text.

In general, the corresponding measurements are taken at 25°C using a Haake RS600 imposed-stress rheometer equipped with a plate-plate measuring body (60 mm diameter) fitted with an anti-evaporation device (bell jar). For each measurement, the sample is placed delicately in position and the measurements start 5 minutes after placing the sample in the jaws (2 mm). The test composition is then subjected to a stress ramp from $10^{-2}$ to $10^{3}$ Pa at a set frequency of 1 Hz.
A composition according to the invention may also have a certain elasticity. This elasticity may be characterized by a stiffness modulus G* which, under this minimum stress threshold, may be at least equal to 400 Pa and preferably greater than 1000 Pa. The value G* of a composition may be obtained by subjecting the composition under consideration to a stress ramp from $10^{-2}$ to $10^{3}$ Pa at a set frequency of 1 Hz.

## HYDROPHILIC GELLING AGENT

For the purposes of the present invention, the term "*hydrophilic gelling agent*" means a compound that is capable of gelling the aqueous phase of the compositions according to the invention.
The gelling agent is hydrophilic and is thus present in the aqueous phase of the composition.
The gelling agent may be water-soluble or water-dispersible. The aqueous phase of a composition according to the present disclosure is gelled with at least one hydrophilic gelling agent chosen from synthetic polymeric gelling agents.
For the purposes of the present disclosure the term "synthetic" means that the polymer is neither naturally existing nor a derivative of a polymer of natural origin.
The synthetic polymeric hydrophilic gelling agent under consideration according to the present disclosure may or may not be particulate.
For the purposes of the invention, the term "particulate" means that the polymer is in the form of particles, preferably spherical particles.
As emerges from the text hereinbelow, the polymeric hydrophilic gelling agent is advantageously chosen from crosslinked acrylic homopolymers or copolymers; associative polymers, in particular associative polymers of polyurethane type; polyacrylamides and crosslinked and/or neutralized 2-acrylamido-2-methylpropanesulfonic acid polymers and copolymers; modified or unmodified carboxyvinyl polymers, and mixtures thereof, especially as defined below.

### A. Particulate synthetic polymeric gelling agents

They are preferably chosen from crosslinked polymers.
They may especially be crosslinked acrylic homopolymers or copolymers, which are preferably partially neutralized or neutralized, and which are in particulate form.
According to one embodiment, the particulate gelling agent according to the present disclosure is chosen from crosslinked sodium polyacrylates. Preferably, it has in the dry or non-hydrated state a mean size of less than or equal to 100 μm and preferably less than or equal to 50 μm. The mean size of the particles corresponds to the mass-average diameter (D50) measured by laser particle size analysis or another equivalent method known to those skilled in the art.
Thus, preferably, the particulate gelling agent according to the present disclosure is chosen from crosslinked sodium polyacrylates, preferably in the form of particles with a mean size (or mean diameter) of less than or equal to 100 microns, more preferably in the form of spherical particles.
As examples of crosslinked sodium polyacrylates, mention may be made of those sold under the brand names Octacare X100, X110 and RM100 by the company Avecia, those sold under the names Flocare GB300 and Flosorb 500 by the company SNF, those sold under the names Luquasorb 1003, Luquasorb 1010, Luquasorb 1280 and Luquasorb 1110 by the company BASF, those sold under the names Water Lock G400 and G430 (INCI name: Acrylamide/Sodium acrylate copolymer) by the company Grain Processing.
Mention may also be made of crosslinked polyacrylate microspheres, for instance those sold under the name Aquakeep® 10 SH NF by the company Sumitomo Seika.
Such gelling agents may be used in a proportion of from 0.1% to 5% by weight of solids relative to the total weight of the aqueous phase, especially from 0.3% to 2% by weight and in particular in a proportion of about from 0.5% to 1.7% by weight, relative to the total weight of the aqueous phase.

### B. Non-particulate synthetic polymeric gelling agents

This family of gelling agents may be detailed under the following subfamilies:

1. Associative polymers,
2. Polyacrylamides and crosslinked and/or neutralized 2-acrylamido-2-methylpropanesulfonic acid polymers and copolymers, and

3. Modified or unmodified carboxyvinyl polymers.

**B.1 Associative polymers**

For the purposes of the present disclosure, the term "*associative polymer*" means any amphiphilic polymer comprising in its structure at least one fatty chain and at least one hydrophilic portion. The associative polymers in accordance with the present disclosure may be anionic, cationic, nonionic or amphoteric.

Associative anionic polymers

Among the associative anionic polymers that may be mentioned are those comprising at least one hydrophilic unit, and at least one fatty-chain allyl ether unit, more particularly those whose hydrophilic unit is formed by an unsaturated ethylenic anionic monomer, more particularly by a vinylcarboxylic acid and most particularly by an acrylic acid or a methacrylic acid or mixtures thereof, and whose fatty-chain allyl ether unit corresponds to the monomer of formula (I) below:

$$CH_2 = C(R')CH_2OB_nR \qquad (I)$$

in which R' denotes H or $CH_3$, B denotes the ethylenoxy radical, n is zero or denotes an integer ranging from 1 to 100, R denotes a hydrocarbon-based radical chosen from alkyl, arylalkyl, aryl, alkylaryl and cycloalkyl radicals, comprising from 8 to 30 carbon atoms, preferably from 10 to 24 and even more particularly from 12 to 18 carbon atoms.

Anionic amphiphilic polymers of this type are described and prepared, according to an emulsion polymerization process, in patent EP 0 216 479.

Among the associative anionic polymers that may also be mentioned are maleic anhydride/$C_{30}$-$C_{38}$-$\alpha$-olefin/alkyl maleate terpolymers, such as the product (maleic anhydride/$C_{30}$-$C_{38}$-$\alpha$-olefin/isopropyl maleate copolymer) sold under the name Performa V 1608 by the company Newphase Technologies.

Among the associative anionic polymers, mention may be made, according to a preferred embodiment, of copolymers comprising among their monomers an $\alpha,\beta$-monoethylenically unsaturated carboxylic acid and an ester of an $\alpha,\beta$-monoethylenically unsaturated carboxylic acid and of an oxyalkylenated fatty alcohol.

Preferentially, these compounds also comprise as monomer an ester of an $\alpha,\beta$-monoethylenically unsaturated carboxylic acid and of a $C_1$-$C_4$ alcohol.

Examples of compounds of this type that may be mentioned include Aculyn 22® sold by the company Röhm & Haas, which is a methacrylic acid/ethyl acrylate/oxyalkylenated stearyl methacrylate (comprising 20 EO units) terpolymer or Aculyn 28® (methacrylic acid/ethyl acrylate/oxyethylenated behenyl methacrylate (25 EO) terpolymer).

Associative anionic polymers that may also be mentioned include anionic polymers comprising at least one hydrophilic unit of unsaturated olefinic carboxylic acid type, and at least one hydrophobic unit exclusively of the type such as a ($C_{10}$-$C_{30}$) alkyl ester of an unsaturated carboxylic acid. Examples that may be mentioned include the anionic polymers described and prepared according to patents US 3 915 921 and US 4 509 949.

Associative anionic polymers that may also be mentioned include anionic terpolymers

The anionic terpolymer used according to the present disclosure is a linear or branched and/or crosslinked terpolymer, of at least one monomer (1) bearing an acid function in free form, which is partially or totally salified with a nonionic monomer (2) chosen from N,N-dimethylacrylamide and 2-hydroxyethyl acrylate and at least one polyoxyethylenated alkyl acrylate monomer (3) of formula (I) below:

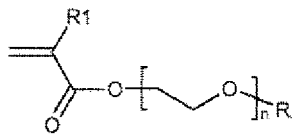

(I)

in which R1 represents a hydrogen atom, R represents a linear or branched $C_2$-$C_8$ alkyl radical and n represents a number ranging from 1 to 10.

The term *"branched polymer"* denotes a non-linear polymer which bears pendent chains so as to obtain, when this polymer is dissolved in water, a high degree of entanglement leading to very high viscosities, at a low speed gradient.

The term *"crosslinked polymer"* denotes a non-linear polymer which is in the form of a three-dimensional network that is insoluble in water but swellable in water, leading to the production of a chemical gel.

The acid function of the monomer (1) is especially a sulfonic acid or phosphonic acid function, said functions

being in free or partially or totally salified form.

The monomer (1) may be chosen from styrenesulfonic acid, ethylsulfonic acid and 2-methyl-2-[(1-oxo-2-propenyl]amino]-1-propanesulfonic acid (also known as acryloyldimethyl taurate), in free or partially or totally salified form. It is present in the anionic terpolymer preferably in molar proportions of between 5 mol% and 95 mol% and more particularly between 10 mol% and 90 mol%. The monomer (1) will more particularly be 2-methyl-2-[(1-oxo-2-propenyl)amino]-1-propanesulfonic acid in free or partially or totally salified form.

The acid function in partially or totally salified form will preferably be an alkali metal salt such as a sodium or potassium salt, an ammonium salt, an amino alcohol salt such as a monoethanolamine salt, or an amino acid salt such as a lysine salt.

The monomer (2) is preferably present in the anionic terpolymer in molar proportions of between 4.9 mol% and 90 mol%, more particularly between 9.5 mol% and 85 mol% and even more particularly between 19.5 mol% and 75 mol%.

In formula (I), examples of linear $C_8$-$C_{16}$ alkyl radicals that may be mentioned include octyl, decyl, undecyl, tridecyl, tetradecyl, pentadecyl and hexadecyl.

In formula (I), examples of branched $C_8$-$C_{16}$ alkyl radicals that may be mentioned include 2-ethylhexyl, 2-propylheptyl, 2-butyloctyl, 2-pentylnonyl, 2-hexyldecyl, 4-methylpentyl, 5-methylhexyl, 6-methylheptyl, 15-methylpentadecyl, 16-methylheptadecyl and 2-hexyloctyl.

According to a particular form of the present disclosure in formula (I), R denotes a $C_{12}$-$C_{16}$ alkyl radical.

According to a particular form of the present disclosure in formula (I), n ranges from 3 to 5.

Tetraethoxylated lauryl acrylate will more particularly be used as monomer of formula (I).

The monomer (3) of formula (I) is preferably present in the anionic terpolymer in molar proportions of between 0.1 mol% and 10 mol% and more particularly between 0.5 mol% and 5 mol%.

According to a particular mode of the present disclosure, the anionic terpolymer is crosslinked and/or branched with a diethylenic or polyethylenic compound in the proportion expressed relative to the total amount of monomers used, from 0.005 mol% to 1 mol%, preferably from 0.01 mol% to 0.5 mol% and more particularly from 0.01 mol% to 0.25 mol%.

The crosslinking agent and/or branching agent is preferably chosen from ethylene glycol dimethacrylate, diallyloxyacetic acid or a salt thereof, such as sodium diallyloxyacetate, tetraallyloxyethane, ethylene glycol diacrylate, diallylurea, triallylamine, trimethylolpropane triacrylate and methylenebis(acrylamide), or mixtures thereof.

The anionic terpolymer may contain additives such as complexing agents, transfer agents or chain-limiting agents.

Use will be made more particularly of an anionic terpolymer of 2-methyl-2-[(1-oxo-2-propenyl)amino]-1-propanesulfonic acid partially or totally salified in the form of the ammonium salt, N,N-dimethylacrylamide and tetraethoxylated lauryl acrylate crosslinked with trimethylolpropane triacrylate, of INCI name Polyacrylate Crosspolymer-6, such as the product sold under the trade name Sepimax Zen® by the company SEPPIC.

Cationic associative polymers

Cationic associative polymers that may be mentioned include polyacrylates bearing amine side groups.

The polyacrylates bearing quaternized or non-quatemized amine side groups contain, for example, hydrophobic groups of the type such as steareth-20 (polyoxyethylenated (20) stearyl alcohol).

Examples of polyacrylates bearing amino side chains that may be mentioned are the polymers 8781-121B or 9492-103 from the company National Starch.

Nonionic associative polymers

The nonionic associative polymers may be chosen from:

- copolymers of vinylpyrrolidone and of fatty-chain hydrophobic monomers;
- copolymers of $C_1$-$C_6$ alkyl methacrylates or acrylates and of amphiphilic monomers comprising at least one fatty chain;
- copolymers of hydrophilic methacrylates or acrylates and of hydrophobic monomers comprising at least one fatty chain, for instance the polyethylene glycol methacrylate/lauryl methacrylate copolymer;
- associative polyurethanes.

Associative polyurethanes are nonionic block copolymers comprising in the chain both hydrophilic blocks usually of polyoxyethylene nature (the polyurethanes may then be referred to as polyurethane polyethers), and hydrophobic blocks that may be aliphatic sequences alone and/or cycloaliphatic and/or aromatic sequences.

In particular, these polymers comprise at least two hydrocarbon-based lipophilic chains containing from 6 to 30 carbon atoms, separated by a hydrophilic block, the hydrocarbon-based chains possibly being pendent chains or

chains at the end of the hydrophilic block. In particular, it is possible for one or more pendent chains to be envisaged. In addition, the polymer may comprise a hydrocarbon-based chain at one end or at both ends of a hydrophilic block.

Associative polyurethanes may be block polymers, in triblock or multiblock form. The hydrophobic blocks may thus be at each end of the chain (for example: triblock copolymer containing a hydrophilic central block) or distributed both at the ends and in the chain (for example: multiblock copolymer). These polymers may also be graft polymers or star polymers. Preferably, the associative polyurethanes are triblock copolymers in which the hydrophilic block is a polyoxyethylene chain comprising from 50 to 1000 oxyethylene groups. In general, associative polyurethanes comprise a urethane bond between the hydrophilic blocks, whence arises the name.

According to one preferred embodiment, a nonionic associative polymer of polyurethane type is used as gelling agent.

As examples of nonionic fatty-chain polyurethane polyethers that may be used in the present disclosure, it is also possible to use Rheolate® FX 1100 (Steareth-100/PEG 136/HDI (hexamethyl diisocyanate) copolymer), Rheolate® 205 containing a urea function, sold by the company Elementis, or Rheolate® 208, 204 or 212, and also Acrysol® RM 184 or Acrysol® RM 2020.

Mention may also be made of the product Elfacos® T210 containing a $C_{12}$-$C_{14}$ alkyl chain, and the product Elfacos® T212 containing a $C_{16-18}$ alkyl chain (PPG-14 Palmeth-60 Hexyl Dicarbamate), from Akzo.

The product DW 1206B® from Röhm & Haas containing a $C_{20}$ alkyl chain and a urethane bond, sold at a solids content of 20% in water, may also be used.

Use may also be made of solutions or dispersions of these polymers, especially in water or in aqueous-alcoholic medium. Examples of such polymers that may be mentioned are Rheolate® 255, Rheolate® 278 and Rheolate® 244 sold by the company Elementis. The products DW 1206F and DW 1206J sold by the company Röhm & Haas may also be used.

The associative polyurethanes that may be used according to the present disclosure are in particular those described in the article by G. Fonnum, J. Bakke and Fk. Hansen, Colloid Polym. Sci., 271, 380-389 (1993).

Even more particularly, according to the present disclosure use may also be made of an associative polyurethane that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

Such polyurethane polyethers are sold in particular by the company Röhm & Haas under the names Aculyn® 46 and Aculyn® 44. Aculyn® 46 is a polycondensate of polyethylene glycol containing 150 or 180 mol of ethylene oxide, of stearyl alcohol and of methylenebis(4-cyclohexyl isocyanate) (SMDI), at 15% by weight in a matrix of maltodextrin (4%) and water (81%), and Aculyn® 44 is a polycondensate of polyethylene glycol containing 150 or 180 mol of ethylene oxide, of decyl alcohol and of methylenebis(4-cyclohexyl isocyanate) (SMDI), at 35% by weight in a mixture of propylene glycol (39%) and water (26%).

Use may also be made of solutions or dispersions of these polymers, especially in water or in aqueous-alcoholic medium. Examples of such polymers that may be mentioned include SER AD FX1010, SER AD FX1035 and SER AD 1070 from the company Elementis, and Rheolate® 255, Rheolate® 278 and Rheolate® 244 sold by the company Elementis. Use may also be made of the products Aculyn® 44, Aculyn® 46, DW 1206F and DW 1206J, and also Acrysol® RM 184 from the company Röhm & Haas, or alternatively Borchigel LW 44 from the company Borchers, and mixtures thereof.

<u>Amphoteric associative polymers</u>

Among the associative amphoteric polymers of the present disclosure, mention may be made of crosslinked or non-crosslinked, branched or unbranched amphoteric polymers, which may be obtained by copolymerization:

1) of at least one monomer of formula (IVa) or (IVb):

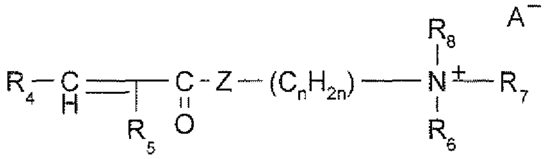

(IVa)

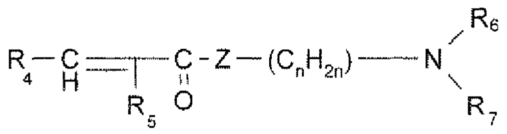

(IVb)

in which $R_4$ and $R_5$, which may be identical or different, represent a hydrogen atom or a methyl radical,
$R_6$, $R_7$ and $R_8$, which may be identical or different, represent a linear or branched alkyl radical containing from 1 to 30 carbon atoms;
Z represents an NH group or an oxygen atom;
n is an integer from 2 to 5;
$A^-$ is an anion derived from a mineral or organic acid, such as a methosulfate anion or a halide such as chloride or bromide;

2) of at least one monomer of formula (V):

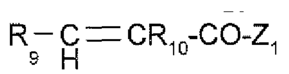

(V)

in which $R_9$ and $R_{10}$, which may be identical or different, represent a hydrogen atom or a methyl radical;
$Z_1$ represents a group OH or a group $NHC(CH_3)_2CH_2SO_3H$;

3) of at least one monomer of formula (VI):

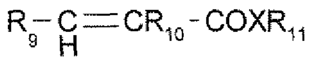

(VI)

in which $R_9$ and $R_{10}$, which may be identical or different, represent a hydrogen atom or a methyl radical, X denotes an oxygen or nitrogen atom and $R_{11}$ denotes a linear or branched alkyl radical containing from 1 to 30 carbon atoms;
4) optionally at least one crosslinking or branching agent; at least one of the monomers of formula (IVa), (IVb) or (VI) comprising at least one fatty chain containing from 8 to 30 carbon atoms and said compounds of the monomers of formulae (IVa), (IVb), (V) and (VI) possibly being quaternized, for example with a $C_1$-$C_4$ alkyl halide or a $C_1$-$C_4$ dialkyl sulfate.

The monomers of formulae (IVa) and (IVb) of the present disclosure are preferably chosen from the group consisting of:

- dimethylaminoethyl methacrylate, dimethylaminoethyl acrylate,
- diethylaminoethyl methacrylate, diethylaminoethyl acrylate,
- dimethylaminopropyl methacrylate, dimethylaminopropyl acrylate,
- dimethylaminopropylmethacrylamide, dimethylaminopropylacrylamide,

which are optionally quaternized, for example with a $C_1$-$C_4$ alkyl halide or a $C_1$-$C_4$ dialkyl sulfate.
More particularly, the monomer of formula (IVa) is chosen from acrylamidopropyltrimethylammonium chloride and methacrylamidopropyltrimethylammonium chloride.
The compounds of formula (V) of the present disclosure are preferably chosen from the group formed by acrylic acid, methacrylic acid, crotonic acid, 2-methylcrotonic acid, 2-acrylamido-2-methylpropanesulfonic acid and 2-methacrylamido-2-methylpropanesulfonic acid. More particularly, the monomer of formula (V) is acrylic acid.
The monomers of formula (VI) of the present disclosure are preferably chosen from the group formed by $C_{12}$-$C_{22}$ and more particularly $C_{16}$-$C_{18}$ alkyl acrylates or methacrylates.
The crosslinking or branching agent is preferably chosen from N,N'-methyienebisacrylamide, triallylmethylammonium chloride, allyl methacrylate, n-methylolacrylamide, polyethylene glycol dimethacrylates, ethylene glycol dimethacrylate, diethylene glycol dimethacrylate, 1,6-hexanediol dimethacrylate and allyl sucrose.
The polymers according to the present disclosure may also contain other monomers such as nonionic monomers and in particular such as $C_1$-$C_4$ alkyl acrylates or methacrylates.
The ratio of the number of cationic charges/anionic charges in these amphoteric polymers is preferably equal to about 1.

The weight-average molecular weights of the associative amphoteric polymers have a weight-average molecular mass of greater than 500, preferably between 10 000 and 10 000 000 and even more preferentially between 100 000 and 8 000 000.

Preferably, the associative amphoteric polymers of the present disclosure contain from 1 mol% to 99 mol%, more preferentially from 20 mol% to 95 mol% and even more preferentially from 25 mol% to 75 mol% of compound(s) of formula (IVa) or (IVb). They also preferably contain from I mol% to 80 mol%, more preferentially from 5 mol% to 80 mol% and even more preferentially from 25 mol% to 75 mol% of compound(s) of formula (V). The content of compound(s) of formula (VI) is preferably between 0.1 mol% and 70 mol%, more preferentially between 1 mol% and 50 mol% and even more preferentially between 1 mol% and 10 mol%. The crosslinking or branching agent, when it is present, is preferably between 0.0001 mol% and 1 mol% and even more preferentially between 0.0001 mol% and 0.1 mol%.

Preferably, the mole ratio between the compound(s) of formula (IVa) or (IVb) and the compound(s) of formula (V) ranges from 20/80 to 95/5 and more preferentially from 25/75 to 75/25.

The associative amphoteric polymers according to the present disclosure are described, for example, in patent application WO 98/44012.

The amphoteric polymers that are particularly preferred according to the present disclosure are chosen from acrylic acid/acrylamidopropyltrimethylammonium chloride/stearyl methacrylate copolymers.

According to a preferred embodiment, the associative polymer is chosen from nonionic associative polymers and more particularly from associative polyurethanes, such as Steareth-100/PLG-136/HDI Copolymer sold under the name Rheolate FX 1100 by Elementis.

Such an associative polymer is advantageously used in a proportion of from 0.1% to 8% by weight of solids and preferably between 0.5% and 4% by weight, relative to the total weight of the aqueous phase.

**B.2 Polyacrylamides and crosslinked and/or neutralized 2-acrylamido-2-methylpropanesulfonic acid polymers and copolymers**

The polymers used that are suitable as aqueous gelling agent for the present disclosure may be crosslinked or non-crosslinked homopolymers or copolymers comprising at least the 2-acrylamidomethylpropanesulfonic acid (AMPS®) monomer, in a form partially or totally neutralized with a mineral base other than aqueous ammonia, such as sodium hydroxide or potassium hydroxide.

They are preferably totally or almost totally neutralized, i.e. at least 90% neutralized.

These AMPS® polymers according to the present disclosure may be crosslinked or non-crosslinked.

When the polymers are crosslinked, the crosslinking agents may be chosen from the polyolefinically unsaturated compounds commonly used for crosslinking polymers obtained by radical polymerization.

Examples of crosslinking agents that may be mentioned include divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allylic or vinyl ethers of polyfunctional alcohols, and also the allylic esters of phosphoric and/or vinylphosphonic acid derivatives, or mixtures of these compounds.

According to one preferred embodiment of the present disclosure, the crosslinking agent is chosen from methylenebisacrylamide, allyl methacrylate and trimethylolpropane triacrylate (TMPTA). The degree of crosslinking generally ranges from 0.01 mol% to 10 mol% and more particularly from 0.2 mol% to 2 mol% relative to the polymer.

The AMPS® polymers that are suitable for use in the present disclosure are water-soluble or water-dispersible. In this case, they are:

- either "homopolymers" comprising only AMPS monomers and, if they are crosslinked, one or more crosslinking agents such as those defined above;
- or copolymers obtained from AMPS® and from one or more hydrophilic or hydrophobic ethylenically unsaturated monomers and, if they are crosslinked, one or more crosslinking agents such as those defined above. When said copolymers comprise hydrophobic ethylenically unsaturated monomers, these monomers do not comprise a fatty chain and are preferably present in small amounts.

For the purpose of the present invention, the term *"fatty chain"* is intended to mean any hydrocarbon-based chain comprising at least 7 carbon atoms.

The term *"water-soluble or water-dispersible"* means polymers which, when introduced into an aqueous phase at 25°C, at a mass concentration equal to 1%, make it possible to obtain a macroscopically homogeneous and transparent solution, i.e. a solution with a maximum light transmittance value, at a wavelength equal to 500 nm, through a sample

1 cm thick, of at least 60% and preferably of at least 70%.

The *"homopolymers"* according to the present disclosure are preferably crosslinked and neutralized, and they may be obtained according to the preparation process comprising the following steps:

(a) the monomer such as AMPS in free form is dispersed or dissolved in a solution of tert-butanol or of water and tert-butanol;
(b) the monomer solution or dispersion obtained in (a) is neutralized with one or more mineral or organic bases, preferably aqueous ammonia NH3, in an amount making it possible to obtain a degree of neutralization of the sulfonic acid functions of the polymer ranging from 90% to 100%;
(c) the crosslinking monomer(s) are added to the solution or dispersion obtained in (b);
(d) a standard free-radical polymerization is performed in the presence of free-radical initiators at a temperature ranging from 10°C to 150°C; the polymer precipitates from the tert-butanol-based solution or dispersion.

The water-soluble or water-dispersible AMPS® copolymers according to the invention contain water-soluble ethylenically unsaturated monomers, hydrophobic monomers, or mixtures thereof.

The water-soluble comonomers may be ionic or nonionic.

Among the ionic water-soluble comonomers, examples that may be mentioned include the following compounds, and salts thereof:

- (meth)acrylic acid,
- styrenesulfonic acid,
- vinyl sulfonic acid and (meth)allylsulfonic acid,
- vinylphosphonic acid,
- maleic acid,
- itaconic acid,
- crotonic acid,
- water-soluble vinyl monomers of formula (A) below:

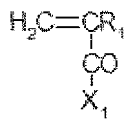

(A)

in which:

- $R_1$ is chosen from H, $-CH_3$, $-C_2H_5$ and $-C_3H_7$,
- $X_1$ is chosen from:
- alkyl oxides of type $-OR_2$ where $R_2$ is a linear or branched, saturated or unsaturated hydrocarbon-based radical containing from 1 to 6 carbon atoms, substituted with at least one sulfonic ($-SO_3-$) and/or sulfate ($-SO_4-$) and/or phosphate ($-PO_4H_2-$) group.

Among the nonionic water-soluble comonomers, examples that may be mentioned include:

- (meth)acrylamide,
- N-vinylacetamide and N-methyl-N-vinylacetamide,
- N-vinylformamide and N-methyl-N-vinylfonnamide,
- maleic anhydride,
- vinylamine,
- N-vinyllactams comprising a cyclic alkyl group containing from 4 to 9 carbon atoms, such as N-vinylpyrrolidone, N-butyrolactam and N-vinylcaprolactam,
- vinyl alcohol of formula $CH_2$=CHOH,
- water-soluble vinyl monomers of formula (B) below:

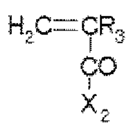

(B)

in which:

- $R_3$ is chosen from H, $-CH_3$, $-C_2H_5$ and $-C_3H_7$,
- $X_2$ is chosen from alkyl oxides of the type $-OR_4$ where $R_4$ is a linear or branched, saturated or unsaturated hydrocarbon-based radical containing from 1 to 6 carbon atoms, optionally substituted with a halogen (iodine, bromine, chlorine or fluorine) atom; a hydroxyl (-OH) group; ether.

Mention is made, for example, of glycidyl (meth)acrylate, hydroxyethyl methacrylate, and (meth)acrylates of ethylene glycol, of diethylene glycol or of polyalkylene glycol.

Among the hydrophobic co-monomers without a fatty chain, mention may be made, for example, of:

- styrene and derivatives thereof, such as 4-butylstyrene, α-methylstyrene and vinyltoluene;
- vinyl acetate of formula $CH_2=CH-OCOCH_3$;
- vinyl ethers of formula $CH_2=CHOR$ in which R is a linear or branched, saturated or unsaturated hydrocarbon-based radical containing from 1 to 6 carbons;
- acrylonitrile;
- caprolactone;
- vinyl chloride and vinylidene chloride;
- silicone derivatives, which, after polymerization, result in silicone polymers such as methacryloxypropyltris(trimethylsiloxy)silane and silicone methacrylamides;
- hydrophobic vinyl monomers of formula (C) below:

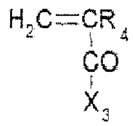
(C)

in which:

- $R_4$ is chosen from H, $-CH_3$, $-C_2H_5$ and $-C_3H_7$;
- $X_3$ is chosen from:
- alkyl oxides of the type $-OR_5$ where $R_5$ is a linear or branched, saturated or unsaturated hydrocarbon-based radical containing from 1 to 6 carbon atoms.

Mention is made, for example, of methyl methacrylate, ethyl methacrylate, n-butyl (meth)acrylate, tert-butyl (meth)acrylate, cyclohexyl acrylate, isobornyl acrylate and 2-ethylhexyl acrylate.

The water-soluble or water-dispersible AMPS® polymers of the invention preferably have a molar mass ranging from 50 000 g/mol to 10 000 000 g/mol, preferably from 80 000 g/mol to 8 000 000 g/mol, and even more preferably from 100 000 g/mol to 7 000 000 g/mol.

As water-soluble or water-dispersible AMPS homopolymers suitable for use in the present disclosure, mention may be made, for example, of crosslinked or non-crosslinked polymers of sodium acrylamido-2-methylpropanesulfonate, such as that used in the commercial product Simulgel 800 (CTFA name: Sodium Polyacryloyldimethyl Taurate), crosslinked ammonium acrylamido-2-methylpropanesulfonate polymers (INCI name: Ammonium Polydimethyltauramide) such as those described in patent EP 0 815 928 B1 and such as the product sold under the trade name Hostacerin AMPS® by the company Clariant.

As water-soluble or water-dispersible AMPS copolymers in accordance with the invention, examples that may be mentioned include:

- crosslinked acrylamide/sodium acrylamido-2-methylpropanesulfonate copolymers, such as that used in the commercial product Sepigel 305® (CTFA name: Polyacrylamide/$C_{13}$-$C_{14}$ Isoparaffin/ Laureth-7) or that used in the commercial product sold under the name Simulgel 600 (CTFA name: Acrylamide/Sodium acryloyldimethyltaurate/Isohexadecane/Polysorbate-80) by the company SEPPIC;
- copolymers of AMPS® and of vinylpyrrolidone or vinylformamide, such as that used in the commercial product sold under the name Aristoflex AVC® by the company Clariant (CTFA name: Ammonium Acryloyldimethyltaurate/VP copolymer) but neutralized with sodium hydroxide or potassium hydroxide;
- copolymers of AMPS® and of sodium acrylate, for instance the AMPS/sodium acrylate copolymer, such as that used in the commercial product sold under the name Simulgel EG® by the company SEPPIC or under the trade name Sepinov EM (CTFA name: Hydroxyethyl acrylate/Sodium acryloyldimethyltaurate copolymer);

- copolymers of AMPS® and of hydroxyethyl acrylate, for instance the AMPS®/hydroxyethyl acrylate copolymer, such as that used in the commercial product sold under the name Simulgel NS® by the company SEPPIC (CTFA name: Hydroxyethyl acrylate/Sodium acryloyldimethyltaurate copolymer (and) Squalane (and) Polysorbate 60), or such as the product sold under the name Sodium acrylamido-2-methylpropanesulfonate/Hydroxyethyl acrylate copolymer, such as the commercial product Sepinov EMT 10 (INCI name: Hydroxyethyl acrylate/Sodium acryloyldimethyltaurate copolymer).

As preferred water-soluble or water-dispersible AMPS copolymers in accordance with the invention, mention may be made of copolymers of AMPS® and of hydroxyethyl acrylate.
In general, an aqueous phase according to the invention may comprise from 0.1% to 10%, preferably from 0.2% to 8%, more preferentially from 0.5% to 6% and even from 0.5% to 5% by weight of solids of polyacrylamide(s) and/or of crosslinked and/or neutralized 2-acrylamido-2-methylpropanesulfonic acid polymer(s) and copolymer(s) relative to its total weight.

**B.3 Modified or unmodified carboxyvinyl polymers**

The modified or unmodified carboxyvinyl polymers may be copolymers derived from the polymerization of at least one monomer (a) chosen from $\alpha,\beta$-ethylenically unsaturated carboxylic acids or esters thereof, with at least one ethylenically unsaturated monomer (b) comprising a hydrophobic group.
The term *"copolymers"* means both copolymers obtained from two types of monomer and those obtained from more than two types of monomer, such as terpolymers obtained from three types of monomer.
Their chemical structure more particularly comprises at least one hydrophilic unit and at least one hydrophobic unit. The term "hydrophobic group or unit" means a radical with a saturated or unsaturated, linear or branched hydrocarbon-based chain, comprising at least 8 carbon atoms, preferably from 10 to 30 carbon atoms, in particular from 12 to 30 carbon atoms and more preferentially from 18 to 30 carbon atoms.
Preferably, these copolymers are chosen from copolymers derived from the polymerization:

- of at least one monomer of formula (1) below:

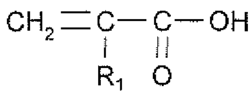

(1)

in which $R_1$ denotes H or $CH_3$ or $C_2H_5$, i.e. acrylic acid, methacrylic acid or ethacrylic acid monomers, and
- of at least one monomer of unsaturated carboxylic acid $(C_{10}-C_{30})$alkyl ester type corresponding to the monomer of formula (2) below:

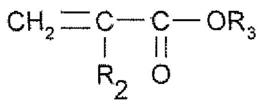

(2)

in which $R_2$ denotes H or $CH_3$ or $C_2H_5$ (i.e. acrylate, methacrylate or ethacrylate units) and preferably H (acrylate units) or $CH_3$ (methacrylate units), $R_3$ denoting a $C_{10}-C_{30}$ and preferably $C_{12}-C_{22}$ alkyl radical.

The unsaturated carboxylic acid $(C_{10}-C_{30})$alkyl esters are preferably chosen from lauryl acrylate, stearyl acrylate, decyl acrylate, isodecyl acrylate and dodecyl acrylate, and the corresponding methacrylates, such as lauryl methacrylate, stearyl methacrylate, decyl methacrylate, isodecyl methacrylate and dodecyl methacrylate, and mixtures thereof.
According to a preferred embodiment, these polymers are crosslinked.
Among the copolymers of this type that will be used more particularly are polymers derived from the polymerization of a monomer mixture comprising:

- essentially acrylic acid,
- an ester of formula (2) described above in which $R_2$ denotes H or $CH_3$, $R_3$ denoting an alkyl radical containing from 12 to 22 carbon atoms, and
- a crosslinking agent, which is a well-known copolymerizable unsaturated polyethylenic monomer, such as diallyl phthalate, allyl (meth)acrylate, divinylbenzene, (poly)ethylene glycol dimethacrylate and methylenebisacrylamide.

Among the copolymers of this type, use will more particularly be made of those consisting of from 95% to 60% by weight of acrylic acid (hydrophilic unit), 4% to 40% by weight of $C_{10}$-$C_{30}$ alkyl acrylate (hydrophobic unit) and 0 to 6% by weight of crosslinking polymerizable monomer, or alternatively those consisting of from 98% to 96% by weight of acrylic acid (hydrophilic unit), 1% to 4% by weight of $C_{10}$-$C_{30}$ alkyl acrylate (hydrophobic unit) and 0.1% to 0.6% by weight of crosslinking polymerizable monomer such as those described previously.

Among the abovementioned polymers, the ones that are most particularly preferred according to the present disclosure are acrylate/$C_{10}$-$C_{30}$-alkyl acrylate copolymers (INCI name: Acrylates/$C_{10-30}$ Alkyl acrylate Crosspolymer) such as the products sold by the company Lubrizol under the trade names Pemulen TR-1, Pemulen TR-2, Carbopol 1382, Carbopol EDT 2020 and Carbopol Ultrez 20 Polymer, and even more preferentially Pemulen TR-2.

Among the modified or unmodified carboxyvinyl polymers, mention may also be made of sodium polyacrylates such as those sold under the name Cosmedia SP® containing 90% solids and 10% water, or Cosmedia SPL® as an inverse emulsion containing about 60% solids, an oil (hydrogenated polydecene) and a surfactant (PPG-5 Laureth-5), both sold by the company Cognis.

Mention may also be made of partially neutralized sodium polyacrylates that are in the form of an inverse emulsion comprising at least one polar oil, for example the product sold under the name Luvigel® EM sold by the company BASF.

The modified or unmodified carboxyvinyl polymers may also be chosen from crosslinked (meth)acrylic acid homopolymers.

For the purposes of the present patent application, the term *"(meth)acrylic"* means *"acrylic or methacrylic"*.

Examples that may be mentioned include the products sold by Lubrizol under the names Carbopol 910, 934, 940, 941, 934 P, 980, 981, 2984, 5984 and Carbopol Ultrez 10 Polymer, or by 3V-Sigma under the name Synthalen® K, Synthalen® L or Synthalen® M.

Among the modified or unmodified carboxyvinyl polymers, mention may be made in particular of Carbopol (INCI name: carbomer) and Pemulen (CTFA name: Acrylates/$C_{10-30}$ alkyl acrylate crosspolymer) sold by the company Lubrizol.

The modified or unmodified carboxyvinyl polymers may be present in a proportion of from 0.1% to 10% by weight of solids relative to the weight of the aqueous phase, in particular from 0.3% to 8% by weight and preferably between 0.4% and 6% by weight, relative to the weight of the aqueous phase.

A composition according to the invention comprises a polymeric hydrophilic gelling agent chosen from crosslinked and/or neutralized 2-acrylamido-2-methylpropanesulfonic acid copolymers.

According to another preferred variant, the synthetic polymeric hydrophilic gelling agent is a copolymer of 2-acrylamido-2-methylpropanesulfonic acid and of hydroxyethyl acrylate.

## LIPOPHILIC GELLING AGENT

For the purposes of the present invention, the term *"lipophilic gelling agent"* means a compound that is capable of gelling the oily phase of the compositions according to the invention.

The gelling agent is lipophilic and is thus present in the oily phase of the composition.

The gelling agent is liposoluble or lipodispersible.

As emerges from the text hereinbelow, the lipophilic gelling agent is advantageously chosen from particulate gelling agents, organopolysiloxane elastomers, semi-crystalline polymers, dextrin esters and polymers containing hydrogen bonding, and mixtures thereof.

### I. Particulate gelling agents

The particulate gelling agent used in the composition according to the present disclosure is in the form of particles, preferably spherical particles.

As representative lipophilic particulate gelling agents that are suitable for use in the present disclosure, mention may be made most particularly of polar and apolar waxes, modified clays, and silicas such as fumed silicas and hydrophobic silica aerogels.

#### Waxes

The term *"wax"* under consideration in the context of the present disclosure generally means a lipophilic compound that is solid at room temperature (25°C), with a solid/liquid reversible change of state, having a melting point of greater than or equal to 30°C, which may be up to 200°C and in particular up to 120°C.

For the purposes of the present disclosure, the melting point corresponds to the temperature of the most endothermic peak observed in thermal analysis (DSC) as described in the standard ISO 11357-3; 1999. The melting point of the wax may be measured using a differential scanning calorimeter (DSC), for example the calorimeter sold

under the name *MDSC 2920* by the company TA Instruments.

The measuring protocol is as follows:

A 5 mg sample of wax placed in a crucible is subjected to a first temperature rise ranging from -20°C to 100°C, at a heating rate of 10°C/minute, it is then cooled from 100°C to -20°C at a cooling rate of 10°C/minute and is finally subjected to a second temperature rise ranging from -20°C to 100°C at a heating rate of 5°C/minute. During the second temperature rise, the variation in the difference in power absorbed by the empty crucible and by the crucible containing the sample of wax is measured as a function of the temperature. The melting point of the compound is the temperature value corresponding to the top of the peak of the curve representing the variation in the difference in power absorbed as a function of the temperature.

The waxes that may be used in the compositions according to the present disclosure are chosen from waxes that are solid at room temperature of animal, vegetable, mineral or synthetic origin, and mixtures thereof.

The waxes, for the purposes of the present disclosure, may be those used generally in the cosmetic or dermatological fields. They may especially be polar or apolar, and hydrocarbon-based, silicone and/or fluoro waxes, optionally comprising ester or hydroxyl functions. They may also be of natural or synthetic origin.

a) Apolar waxes

For the purposes of the present disclosure, the term *"apolar wax"* means a wax whose solubility parameter at 25°C as defined below, $\delta_a$, is equal to 0 $(J/cm^3)^{½}$.

The definition and calculation of the solubility parameters in the Hansen three-dimensional solubility space are described in the article by C.M. Hansen: The three-dimensional solubility parameters, J. Paint Technol. 39, 105 (1967).

According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{½}$.

The parameters $\delta_p$, $\delta_h$, $\delta_D$ and $\delta_a$ are expressed in $(J/cm^3)^{½}$.

Apolar waxes are in particular hydrocarbon-based waxes constituted solely of carbon and hydrogen atoms, and free of heteroatoms such as N, O, Si and P.

The apolar waxes are chosen from microcrystalline waxes, paraffin waxes, ozokerite and polyethylene waxes, and mixtures thereof.

An ozokerite that may be mentioned is Ozokerite Wax SP 1020 P.

As microcrystalline waxes that may be used, mention may be made of Multiwax W 445® sold by the company Sonneborn, and Microwax HW® and Base Wax 30540® sold by the company Paramelt, and Cerewax® No. 3 sold by the company Baerlocher.

As microwaxes that may be used in the compositions according to the present disclosure as apolar wax, mention may be made especially of polyethylene microwaxes such as those sold under the names Micropoly 200®, 220®, 220L® and 250S® by the company Micro Powders.

Polyethylene waxes that may be mentioned include Performalene 500-L Polyethylene and Performalene 400 Polyethylene sold by New Phase Technologies, and Asensa® SC 211 sold by the company Honeywell.

b) Polar wax

For the purposes of the present disclosure, the term *"polar wax"* means a wax whose solubility parameter at 25°C, δa, is other than 0 $(J/cm^3)^{½}$.

In particular, the term *"polar wax"* means a wax whose chemical structure is formed essentially from, or even constituted of, carbon and hydrogen atoms, and that comprises at least one highly electronegative heteroatom such as an oxygen, nitrogen, silicon or phosphorus atom.

The polar waxes may especially be hydrocarbon-based, fluoro or silicone waxes.

Preferentially, the polar waxes may be hydrocarbon-based waxes.

The term *"hydrocarbon-based wax"* is intended to mean a wax formed essentially from, or even constituted of, carbon and hydrogen atoms, and optionally oxygen and nitrogen atoms, and that does not contain any silicon or fluorine atoms. It may contain alcohol, ester, ether, carboxylic acid, amine and/or amide groups.

According to the present disclosure, the term *"ester wax"* means a wax comprising at least one ester function.

According to the invention, the term *"alcohol wax"* means a wax comprising at least one alcohol function, i.e. comprising at least one free hydroxyl (OH) group.

The following may especially be used as ester wax:

- ester waxes such as those chosen from:

  i) waxes of formula $R_1COOR_2$ in which $R_1$ and $R_2$ represent linear, branched or cyclic aliphatic chains in which the number of atoms ranges from 10 to 50, which may contain a heteroatom such as O, N or P and whose melting point ranges from 25 to 120°C,
  ii) bis(1,1,1-trimethylolpropane) tetrastearate, sold under the name Hest 2T-4S® by the company Heterene;
  iii) diester waxes of a dicarboxylic acid of general formula $R^3$-(-OCO-$R^4$-COO-$R^5$), in which $R^3$ and $R^5$ are identical or different, preferably identical, and represent a $C_4$-$C_{30}$ alkyl group (alkyl group comprising from 4 to 30 carbon atoms) and $R^4$ represents a linear or branched $C_4$-$C_{30}$ aliphatic group (alkyl group comprising from 4 to 30 carbon atoms) which may or may not comprise one or more unsaturations and which is preferably linear and unsaturated;
  iv) mention may also be made of the waxes obtained by catalytic hydrogenation of animal or plant oils having linear or branched $C_8$-$C_{32}$ fatty chains, for instance as hydrogenated jojoba oil, hydrogenated sunflower oil, hydrogenated castor oil, hydrogenated coconut oil, and also the waxes obtained by hydrogenation of castor oil esterified with cetyl alcohol;
  v) beeswax, synthetic beeswax, polyglycerolated beeswax, carnauba wax, candelilla wax, oxypropylenated lanolin wax, rice bran wax, ouricury wax, esparto grass wax, cork fibre wax, sugar cane wax, Japan wax, sumach wax, montan wax, orange wax, laurel wax, hydrogenated jojoba wax, sunflower wax, lemon wax, olive wax or berry wax.

According to another embodiment, the polar wax may be an alcohol wax. According to the present disclosure, the term *"alcohol wax"* means a wax comprising at least one alcohol function, i.e. comprising at least one free hydroxyl (OH) group. Alcohol waxes that may be mentioned include for example the $C_{30-50}$ alcohol wax Performacol® 550 Alcohol sold by the company New Phase Technologies, stearyl alcohol and cetyl alcohol.

It is also possible to use silicone waxes, which may advantageously be substituted polysiloxanes, preferably of low melting point.

The term *"silicone wax"* means an oil comprising at least one silicon atom, and especially comprising Si-O groups.

Among the commercial silicone waxes of this type, mention may be made especially of those sold under the names Abilwax 9800, 9801 or 9810 (Goldschmidt), KF910 and KF7002 (Shin-Etsu), or 176-1118-3 and 176-11481 (General Electric).

The silicone waxes that may be used may also be alkyl or alkoxy dimethicones, and also ($C_{20}$-$C_{60}$)alkyl dimethicones, in particular ($C_{30}$-$C_{45}$)alkyl dimethicones, such as the silicone wax sold under the name SF-1642 by the company GE-Bayer Silicones or $C_{30-45}$ alkyl dimethylsilyl polypropylsilsesquioxane sold under the name SW-8005® C30 Resin Wax by the company Dow Corning.

In the context of the present disclosure, particularly advantageous waxes that may be mentioned include polyethylene waxes, jojoba wax, candelilla wax and silicone waxes, in particular candelilla wax.

They may be present in the oily phase in a proportion of from 0.5% to 30% by weight relative to the weight of the oily phase, for example between 5% and 20% of the oily phase and more particularly from 2% to 15% by weight relative to the weight of the oily phase.

Modified clays

The composition according to the present disclosure may comprise at least one lipophilic clay.

The clays may be natural or synthetic, and they are made lipophilic by treatment with an alkylammonium salt such as a $C_{10}$ à $C_{22}$ ammonium chloride, for example distearyldimethylammonium chloride.

They may be chosen from bentonites, in particular hectorites and montmorillonites, beidellites, saponites, nontronites, sepiolites, biotites, attapulgites, vermiculites and zeolites.

They are preferably chosen from hectorites.

Hectorites modified with a $C_{10}$ a $C_{22}$ ammonium chloride, such as hectorite modified with distearyldimethylammonium chloride, for instance the product sold under the name Bentone 38V® by the company Elementis or bentone gel in isododecane sold under the name Bentone Gel ISD V® (87% isododecane/10% disteardimonium hectori.te/3% propylene carbonate) by the company Elementis, are preferably used as lipophilic clays.

Lipophilic clay may especially be present in a content ranging from 0.1% to 15% by weight, particular from 0.5% to 10% and more particularly from 1% to 10% by weight relative to the total weight of the oily phase.

Silicas

The oily phase of a composition according to the present disclosure may also comprise, as gelling agent, a fumed silica or silica aerogel particles.

a) Fumed silica

Fumed silica which has undergone a hydrophobic surface treatment is most particularly suitable for use in the invention. Specifically, it is possible to chemically modify the surface of silica, by chemical reaction generating a reduced number of silanol groups present at the surface of the silica. It is possible in particular to replace silanol groups with hydrophobic groups: a hydrophobic silica is then obtained.

The hydrophobic groups may be:

- trimethylsiloxyl groups, which are obtained in particular by treating fumed silica in the presence of hexamethyldisilazane. Silicas thus treated are known as Silica Silylate according to the CTFA (8$^{th}$ edition, 2000). They are sold, for example, under the references Aerosil R812® by the company Degussa and Cab-O-Sil TS-530® by the company Cabot.
- dimethylsilyloxyl or polydimethylsiloxane groups, which are obtained in particular by treating fumed silica in the presence of polydimethylsiloxane or dimethyldichlorosilane. Silicas thus treated are known as Silica dimethyl silylate according to the CTFA (8th Edition, 2000). They are sold, for example, under the references Aerosil R972® and Aerosil R974® by the company Degussa, and Cab-O-Sil TS-610® and Cab-O-Sil TS-720® by the company Cabot.

The fumed silicas may be present in a composition according to the present disclosure in a content of between 0.1% and 40% by weight, more particularly between 1% and 15% by weight and even more particularly between 2% and 10% by weight relative to the total weight of the oily phase.

b) Hydrophobic silica aerogels

The oily phase of a composition according to the present disclosure may also comprise, as gelling agent, at least silica aerogel particles.

Silica aerogels are porous materials obtained by replacing (by drying) the liquid component of a silica gel with air.

They are generally synthesized via a sol-gel process in a liquid medium and then dried, usually by extraction with a supercritical fluid, the one most commonly used being supercritical $CO_2$. This type of drying makes it possible to avoid shrinkage of the pores and of the material. The sol-gel process and the various drying operations are described in detail in Brinker C.J, and Scherer G.W., Sol-Gel Science, New York: Academic Press, 1990.

The hydrophobic silica aerogel particles used in the present disclosure have a specific surface area per unit mass (SM) ranging from 500 to 1500 $m^2/g$, preferably from 600 to 1200 $m^2/g$ and better still from 600 to 800 $m^2/g$, and a size expressed as the volume-mean diameter (D[0.5]) ranging from 1 to 1500 μm, better still from 1 to 1000 μm, preferably from 1 to 100 μm, in particular from 1 to 30 μm, more preferably from 5 to 25 μm, better still from 5 to 20 μm and even better still from 5 to 15 μm.

According to one embodiment, the hydrophobic silica aerogel particles used in the present invention have a size expressed as volume-mean diameter (D[0.5]) ranging from 1 to 30 μm, preferably from 5 to 25 μm, better still from 5 to 20 μm and even better still from 5 to 15 μm.

The specific surface area per unit mass may be determined by the nitrogen absorption method, known as the BET (Brunauer-Emmett-Teller) method, described in the Journal of the American Chemical Society, vol. 60, page 309, February 1938, which corresponds to International Standard ISO 5794/1 (appendix D). The BET specific surface area corresponds to the total specific surface area of the particles under consideration.

The sizes of the silica aerogel particles may be measured by static light scattering using a commercial particle size analyser such as the MasterSizer 2000 machine from Malvern. The data are processed on the basis of the Mie scattering theory. This theory, which is exact for isotropic particles, makes it possible to determine, in the case of non-spherical particles, an "effective" particle diameter. This theory is especially described in the publication by Van de Hulst, H.C., Light Scattering by Small Particles, Chapters 9 and 10, Wiley, New York, 1957.

According to an advantageous embodiment, the hydrophobic silica aerogel particles used in the present invention have a specific surface area per unit of mass (SM) ranging from 600 to 800 $m^2/g$.

The silica aerogel particles used in the present disclosure may advantageously have a tapped density ρ ranging from 0.02 $g/cm^3$ to 0.10 $g/cm^3$, preferably from 0.03 $g/cm^3$ to 0.08 $g/cm^3$ and in particular ranging from 0.05 $g/cm^3$ to 0.08 $g/cm^3$.

In the context of the present disclosure, this density, known as the tapped density, may be assessed according

to the following protocol:

40 g of powder are poured into a measuring cylinder; the measuring cylinder is then placed on a Stav 2003 machine from Stampf Volumeter; the measuring cylinder is then subjected to a series of 2500 tapping actions (this operation is repeated until the difference in volume between two consecutive tests is less than 2%); the final volume Vf of tapped powder is then measured directly on the measuring cylinder. The tapped density is determined by the ratio m/Vf, in this instance 40/Vf (Vf being expressed in $cm^3$ and m in g).

According to one preferred embodiment, the hydrophobic silica aerogel particles used in the present disclosure have a specific surface area per unit of volume SV ranging from 5 to 60 $m^2/cm^3$, preferably from 10 to 50 $m^2/cm^3$ and better still from 15 to 40 $m^2/cm^3$.

The specific surface area per unit volume is given by the relationship: $S_V = S_M \times \rho$;, where ρ is the tapped density, expressed in $g/cm^3$, and $S_M$ is the specific surface area per unit of mass, expressed in $m^2/g$, as defined above.

Preferably, the hydrophobic silica aerogel particles according to the present disclosure have an oil-absorbing capacity, measured at the wet point, ranging from 5 to 18 ml/g, preferably from 6 to 15 ml/g and better still from 8 to 12 ml/g.

The absorbing capacity measured at the wet point, noted Wp, corresponds to the amount of oil that needs to be added to 100 g of particles in order to obtain a homogeneous paste.

It is measured according to the wet point method or the method for determining the oil uptake of a powder described in standard NF T 30-022. It corresponds to the amount of oil adsorbed onto the available surface of the powder and/or absorbed by the powder by measurement of the wet point, described below:
An amount m = 2 g of powder is placed on a glass plate, and the oil (isononyl isononanoate) is then added dropwise. After addition of 4 to 5 drops of oil to the powder, mixing is carried out using a spatula, and addition of oil is continued until conglomerates of oil and powder have formed. From this point, the oil is added at the rate of one drop at a time and the mixture is subsequently triturated with the spatula. The addition of oil is stopped when a firm, smooth paste is obtained. This paste must be able to be spread on the glass plate without cracking or forming lumps. The volume Vs (expressed in ml) of oil used is then noted.

The oil uptake corresponds to the ratio Vs/m.

The aerogels used according to the present disclosure are aerogels of hydrophobic silica, preferably of silylated silica (INCI name: silica silylate).

The term "*hydrophobic silica*" means any silica whose surface is treated with silylating agents, for example with halogenated silanes such as alkylchlorosilanes, siloxanes, in particular dimethylsiloxanes such as hexamethyldisiloxane, or silazanes, so as to functionalize the OH groups with silyl groups Si-Rn, for example trimethylsilyl groups.

As regards the preparation of hydrophobic silica aerogel particles that have been surface-modified by silylation, reference may be made to document US 7 470 725.

Use will preferably be made of hydrophobic silica aerogel particles surface-modified with trimethylsilyl groups, preferably of the INCI name Silica silylate.

As hydrophobic silica aerogels that may be used in the present disclosure, an example that may be mentioned is the aerogel sold under the name VM-2260 or VM-2270 (INCI name: Silica silylate) by the company Dow Corning, the particles of which have a mean size of about 1000 microns and a specific surface area per unit of mass ranging from 600 to 800 $m^2/g$.

Mention may also be made of the aerogels sold by the company Cabot under the references Aerogel TLD 201, Aerogel OGD 201 and Aerogel TLD 203, Enova® Aerogel MT 1100 and Enova Aerogel MT 1200.

Use will preferably be made of the aerogel sold under the name VM-2270 (INCI name: Silica silylate) by the company Dow Corning, the particles of which have an average size ranging from 5-15 microns and a specific surface area per unit of mass ranging from 600 to 800 $m^2/g$.

Preferably, the hydrophobic silica aerogel particles are present in the composition according to the present disclosure in a solids content ranging from 0.1% to 8% by weight, preferably from 0.2% to 5% by weight and preferably from 0.2% to 3% by weight relative to the total weight of the oily phase.

## II. Organopolysiloxane elastomer

The organopolysiloxane elastomer used as lipophilic gelling agent has the advantage of giving the composition according to the invention good application properties. It affords a very soft feel and a matt effect after application, which is advantageous especially for application to the skin. It may also allow efficient filling of the hollows present on keratin materials.

The term "*organopolysiloxane elastomer*" or "*silicone elastomer*" means a supple, deformable organopolysiloxane with viscoelastic properties and especially with the consistency of a sponge or a supple sphere. Its modulus of elasticity is such that this material withstands deformation and has a limited ability to extend and to contract. This material is capable of regaining its original shape after stretching.

It is more particularly a crosslinked organopolysiloxane elastomer.

Thus, the organopolysiloxane elastomer may be obtained by crosslinking addition reaction of diorganopolysiloxane containing at least one hydrogen bonded to silicon and of diorganopolysiloxane containing ethylenically unsaturated groups bonded to silicon, especially in the presence of a platinum catalyst; or by dehydrogenation crosslinking condensation reaction between a diorganopolysiloxane comprising hydroxyl end groups and a diorganopolysiloxane containing at least one hydrogen bonded to silicon, especially in the presence of an organotin; or by crosslinking condensation reaction of a diorganopolysiloxane comprising hydroxyl end groups and of a hydrolysable organopolysilane; or by thermal crosslinking of organopolysiloxane, especially in the presence of an organoperoxide catalyst; or by crosslinking of organopolysiloxane via high-energy radiation such as gamma rays, ultraviolet rays or an electron beam.

Preferably, the organopolysiloxane elastomer is obtained by crosslinking addition reaction (A) of diorganopolysiloxane containing at least two hydrogens each bonded to a silicon, and (B) of diorganopolysiloxane containing at least two ethylenically unsaturated groups bonded to silicon, especially in the presence (C) of a platinum catalyst, as described, for instance, in patent application EP-A-295 886.

In particular, the organopolysiloxane elastomer may be obtained by reaction of dimethylpolysiloxane comprising dimethylvinylsiloxy end groups and of methylhydrogenopolysiloxane comprising trimethylsiloxy end groups, in the presence of a platinum catalyst.

Compound (A) is the base reagent for the formation of organopolysiloxane elastomer, and the crosslinking is performed by addition reaction of compound (A) with compound (B) in the presence of the catalyst (C).

Compound (A) is in particular an organopolysiloxane containing at least two hydrogen atoms bonded to different silicon atoms in each molecule.

Compound (A) may have any molecular structure, in particular a linear-chain or branched-chain structure or a cyclic structure.

Compound (A) may have a viscosity at 25°C ranging from 1 to 50 000 centistokes, in particular in order to be satisfactorily miscible with compound (B).

The organic groups bonded to the silicon atoms of compound (A) may be alkyl groups such as methyl, ethyl, propyl, butyl, octyl; substituted alkyl groups such as 2-phenylethyl, 2-phenylpropyl or 3,3,3-trifluoropropyl; aryl groups such as phenyl, tolyl, xylyl; substituted aryl groups such as phenylethyl; and substituted monovalent hydrocarbon-based groups such as an epoxy group, a carboxylate ester group or a mercapto group.

Compound (A) can thus be chosen from methylhydropolysiloxanes comprising trimethylsiloxy end groups, dimethylsiloxane-methylhydrosiloxane copolymers comprising trimethylsiloxy end groups, and dimethylsiloxane-methylhydrosiloxane cyclic copolymers.

Compound (B) is advantageously a diorganopolysiloxane containing at least two lower alkenyl groups (for example $C_2$-$C_4$); the lower alkenyl group may be chosen from vinyl, allyl and propenyl groups. These lower alkenyl groups may be located at any position on the organopolysiloxane molecule but are preferably located at the ends of the organopolysiloxane molecule. The organopolysiloxane (B) may have a branched-chain, linear-chain, cyclic or network structure but the linear-chain structure is preferred. Compound (B) may have a viscosity ranging from the liquid state to the gum state. Preferably, compound (B) has a viscosity of at least 100 centistokes at 25°C.

Besides the abovementioned alkenyl groups, the other organic groups bonded to the silicon atoms in compound (B) may be alkyl groups such as methyl, ethyl, propyl, butyl or octyl; substituted alkyl groups such as 2-phenylethyl, 2-phenylpropyl or 3,3,3-trifluoropropyl; aryl groups such as phenyl, tolyl or xylyl; substituted aryl groups such as phenylethyl; and substituted monovalent hydrocarbon-based groups such as an epoxy group, a carboxylate ester group or a mercapto group.

The organopolysiloxanes (B) can be chosen from methylvinylpolysiloxanes, methylvinylsiloxane-dimethylsiloxane copolymers, dimethylpolysiloxanes comprising dimethylvinylsiloxy end groups, dimethylsiloxane-methylphenylsiloxane copolymers comprising dimethylvinylsiloxy end groups, dimethylsiloxane-diphenylsiloxane-methylvinylsiloxane copolymers comprising dimethylvinylsiloxy end groups, dimethylsiloxane-methylvinylsiloxane copolymers comprising trimethylsiloxy end groups, dimethylsiloxane-methylphenylsiloxane-methylvinylsiloxane copolymers comprising trimethylsiloxy end groups, methyl(3,3,3-trifluoropropyl)polysiloxanes comprising dimethylvinylsiloxy end groups, and dimethylsiloxane-methyl(3,3,3-trifluoropropyl)siloxane copolymers comprising dimethylvinylsiloxy end groups.

In particular, the organopolysiloxane elastomer can be obtained by reaction of dimethylpolysiloxane comprising dimethylvinylsiloxy end groups and of methylhydropolysiloxane comprising trimethylsiloxy end groups, in the presence of a platinum catalyst.

Advantageously, the sum of the number of ethylenic groups per molecule of compound (B) and of the number of hydrogen atoms bonded to silicon atoms per molecule of compound (A) is at least 5.

It is advantageous for compound (A) to be added in an amount such that the molecular ratio of the total amount of hydrogen atoms bonded to silicon atoms in compound (A) to the total amount of all the ethylenically unsaturated groups in compound (B) is within the range from 1.5/1 to 20/1.

Compound (C) is the catalyst for the crosslinking reaction, and is especially chloroplatinic acid, chloroplatinic acid-olefin complexes, chloroplatinic acid-alkenylsiloxane complexes, chloroplatinic acid-diketone complexes, platinum

black and platinum on a support.

Catalyst (C) is preferably added in an amount of from 0.1 to 1000 parts by weight and better still from 1 to 100 parts by weight, as clean platinum metal, per 1000 parts by weight of the total amount of compounds (A) and (B).

The elastomer is advantageously a non-emulsifying elastomer.

The term "*non-emulsifying*" defines organopolysiloxane elastomers not containing any hydrophilic chains, and in particular not containing any polyoxyalkylene units (especially polyoxyethylene or polyoxypropylene) or any polyglyceryl units. Thus, according to one particular mode of the invention, the composition comprises an organopolysiloxane elastomer free of polyoxyalkylene units and of polyglyceryl units.

In particular, the silicone elastomer used in the present invention is chosen from Dimethicone Crosspolymer (INCI name), Vinyl Dimethicone Crosspolymer (INCI name), Dimethicone/Vinyl Dimethicone Crosspolymer (INCI name), Dimethicone Crosspolymer-3 (INCI name).

The organopolysiloxane elastomer particles may be conveyed in the form of a gel formed from an elastomeric organopolysiloxane included in at least one hydrocarbon-based oil and/or one silicone oil. In these gels, the organopolysiloxane particles are often non-spherical particles.

Non-emulsifying elastomers are described especially in patents EP 242 219, EP 285 886 and EP 765 656 and in patent application JP-A-61-194009.

The silicone elastomer is generally in the form of a gel, a paste or a powder, but advantageously in the form of a gel in which the silicone elastomer is dispersed in a linear silicone oil (dimethicone) or cyclic silicone oil (e.g.: cyclopentasiloxane), advantageously in a linear silicone oil.

Non-emulsifying elastomers that may be used more particularly include those sold under the names KSG-6, KSG-15, KSG-16, KSG-18, KSG-41, KSG-42, KSG-43 and KSG-44 by the company Shin-Etsu, DC9040 and DC9041 by the company Dow Corning, and SFE 839 by the company General Electric.

According to a particular mode, use is made of a gel of silicone elastomer dispersed in a silicone oil chosen from a non-exhaustive list comprising cyclopentadimethylsiloxane, dimethicones, dimethylsiloxanes, methyl trimethicone, phenyl methicone, phenyl dimethicone, phenyl trimethicone and cyclomethicone, preferably a linear silicone oil chosen from polydimethylsiloxanes (PDMS) or dimethicones with a viscosity at 25°C ranging from 1 to 500 cSt, optionally modified with optionally fluorinated aliphatic groups, or with functional groups such as hydroxyl, thiol and/or amine groups.

Mention may be made especially of the compounds having the following INCI names:

- dimethicone/vinyl dimethicone crosspolymer, such as USG-105 and USG-107A from the company Shin-Etsu; DC9506 and DC9701 from the company Dow Corning;
- dimethicone/vinyl dimethicone crosspolymer (and) dimethicone, such as KSG-6 and KSG-16 from the company Shin-Etsu;
- dimethicone/vinyl dimethicone crosspolymer (and) cyclopentasiloxane, such as KSG-15;
- cyclopentasiloxane (and) dimethicone crosspolymer, such as DC9040, DC9045 and DC5930 from the company Dow Corning;
- dimethicone (and) dimethicone crosspolymer, such as DC9041 from the company Dow Corning.
- dimethicone (and) dimethicone crosspolymer, such as Dow Corning EL-9240® Silicone Elastomer Blend from the company Dow Corning (mixture of polydimethylsiloxane crosslinked with hexadiene/polydimethylsiloxane (2 cSt));
- $C_{4-24}$ alkyl dimethicone/divinyl dimethicone crosspolymer, such as NuLastic Silk MA from the company Alzo.

As examples of silicone elastomers dispersed in a linear silicone oil that may advantageously be used according to the invention, mention may especially be made of the following references:

- dimethicone/vinyl dimethicone crosspolymer (and) dimethicone, such as KSG-6 and KSG-16 from the company Shin-Etsu;
- dimethicone (and) dimethicone crosspolymer, such as DC9041 from the company Dow Corning; and
- dimethicone (and) dimethicone crosspolymer, such as Dow Corning EL-9240® Silicone Elastomer Blend from the company Dow Corning (mixture of polydimethylsiloxane crosslinked with hexadiene/polydimethylsiloxane (2 cSt)).

According to a preferred embodiment, the composition according to the invention comprises at least one crosslinked silicone elastomer having the INCI name "dimethicone crosspolymer" or "dimethicone (and) dimethicone crosspolymer", with, preferably, a dimethicone having a viscosity ranging from 1 to 100 cSt, in particular from 1 to 10 cSt at 25°C, such as the mixture of polydimethylsiloxane crosslinked with hexadiene/polydimethylsiloxane (5 cSt) sold under the name DC 9041 by the company Dow Corning or the mixture of polydimethylsiloxane crosslinked with hexadiene/polydimethylsiloxane (2 cSt) sold under the name EL-9240® by the company Dow Corning.

According to a particularly preferred embodiment, the composition according to the invention comprises at least one crosslinked silicone elastomer having the INCI name "dimethicone (and) dimethicone crosspolymer", preferably

with a dimethicone having a viscosity ranging from 1 to 100 cSt, in particular from 1 to 10 cSt at 25°C, such as the mixture of polydimethylsiloxane crosslinked with hexadiene/polydimethylsiloxane (5 cSt) sold under the name DC 9041 by the company Dow Corning.

The organopolysiloxane elastomer particles may also be used in powder form: mention may be made especially of the powders sold under the names Dow Corning 9505 Powder and Dow Corning 9506 Powder by the company Dow Corning, these powders having the INCI name: dimethicone/vinyl dimethicone crosspolymer.

The organopolysiloxane powder may also be coated with silsesquioxane resin, as described, for example, in patent US 5 538 793. Such elastomeric powders are sold under the names KSP-100, KSP-101, KSP-102, KSP-103, KSP-104 and KSP-105 by the company Shin-Etsu, and have the INCI name: vinyl dimethicone/methicone silsesquioxane crosspolymer.

As examples of organopolysiloxane powders coated with silsesquioxane resin that may advantageously be used according to the invention, mention may be made especially of the reference KSP-100 from the company Shin-Etsu.

According to a particularly preferred mode, the composition according to the invention comprises at least one crosslinked silicone elastomer having the INCI name: vinyl dimethicone/methicone silsesquioxane crosspolymer, as oily gelling agent and/or as soft-focus filler.

As preferred lipophilic gelling agent of organopolysiloxane elastomer type, mention may be made especially of crosslinked organopolysiloxane elastomers chosen from Dimethicone Crosspolymer (INCI name), Dimethicone (and) Dimethicone Crosspolymer (INCI name), Vinyl Dimethicone Crosspolymer (INCI name), Dimethicone/Vinyl Dimethicone Crosspolymer (INCI name), Dimethicone Crosspolymer-3 (INCI name), and in particular Dimethicone Crosspolymer (INCI name).

The organopolysiloxane elastomer may be present in a composition according to the present invention in a content of between 0.2% and 10% by weight of active material (solids) and especially between 0.2% and 5% by weight relative to the total weight of the oily phase.

## III. Semi-crystalline polymers

The composition according to the present disclosure may comprise at least one semi-crystalline polymer. Preferably, the semi-crystalline polymer has an organic structure, and a melting point of greater than or equal to 30°C.

For the purposes of the present disclosure, the term *"semi-crystalline polymer"* means polymers comprising a crystallizable portion and an amorphous portion and having a first-order reversible change of phase temperature, in particular of melting point (solid-liquid transition). The crystallizable portion is either a side chain (or pendent chain) or a block in the backbone.

When the crystallizable portion of the semi-crystalline polymer is a block of the polymer backbone, this crystallizable block has a chemical nature different than that of the amorphous blocks; in this case, the semi-crystalline polymer is a block copolymer, for example of the diblock, triblock or multiblock type. When the crystallizable portion is a chain that is pendent on the backbone, the semicrystalline polymer may be a homopolymer or a copolymer.

The melting point of the semi-crystalline polymer is preferably less than 150°C.

The melting point of the semi-crystalline polymer is preferably greater than or equal to 30°C and less than 100°C. More preferably, the melting point of the semi-crystalline polymer is greater than or equal to 30°C and less than 70°C.

The semi-crystalline polymer(s) according to the present disclosure are solid at room temperature (25°C) and atmospheric pressure (760 mmHg), with a melting point of greater than or equal to 30°C. The melting point values correspond to the melting point measured using a differential scanning calorimeter (DSC), such as the calorimeter sold under the name DSC 30 by the company Mettler, with a temperature rise of 5 or 10°C per minute (the melting point under consideration is the point corresponding to the temperature of the most endothermic peak in the thermogram).

The semi-crystalline polymer(s) according to the present disclosure preferably have a melting point that is higher than the temperature of the keratin support intended to receive said composition, in particular the skin or the lips.

According to the present disclosure, the semi-crystalline polymers are advantageously soluble in the fatty phase, especially to at least 1% by weight, at a temperature that is higher than their melting point. Besides the crystallizable chains or blocks, the blocks of the polymers are amorphous.

For the purposes of the present disclosure, the term *"crystallizable chain or block"* means a chain or block which, if it were alone, would change from the amorphous state to the crystalline state reversibly, depending on whether the temperature is above or below the melting point. For the purposes of the present disclosure, a "chain" is a group of atoms, which are pendent or lateral relative to the polymer backbone. A "block" is a group of atoms belonging to the backbone, this group constituting one of the repeating units of the polymer.

Preferably, the polymer backbone of the semi-crystalline polymers is soluble in the fatty phase at a temperature above their melting point.

Preferably, the crystallizable blocks or chains of the semi-crystalline polymers represent at least 30% of the

total weight of each polymer and better still at least 40%. The semi-crystalline polymers containing crystallizable side chains are homopolymers or copolymers. The semi-crystalline polymers of the invention containing crystallizable blocks are block or multiblock copolymers. They may be obtained via polymerization of a monomer containing reactive double bonds (or ethylenic double bonds) or via polycondensation. When the polymers of the invention are polymers containing crystallizable side chains, these side chains are advantageously in random or statistical form.

Preferably, the semi-crystalline polymers of the invention are of synthetic origin.

According to a preferred embodiment, the semi-crystalline polymer is chosen from:

- homopolymers and copolymers comprising units resulting from the polymerization of one or more monomers bearing crystallizable hydrophobic side chain(s),
- polymers bearing in the backbone at least one crystallizable block,
- polycondensates of aliphatic or aromatic or aliphatic/aromatic polyester type,
- copolymers of ethylene and propylene prepared via metallocene catalysis, and
- acrylate/silicone copolymers.

The semi-crystalline polymers that may be used in the present disclosure may be chosen in particular from:

- block copolymers of polyolefins of controlled crystallization, whose monomers are described in EP-A-0 951 897,
- polycondensates, especially of aliphatic or aromatic or aliphatic/aromatic polyester type,
- copolymers of ethylene and propylene prepared via metallocene catalysis,
- homopolymers or copolymers bearing at least one crystallizable side chain and homopolymers or copolymers bearing in the backbone at least one crystallizable block, such as those described in document US-A-5 156 911, such as the ($C_{10}$-$C_{30}$)alkyl polyacrylates corresponding to the Intelimer® products from the company Landec described in the brochure *Intelimer® Polymers*, Landec IP22 (Rev. 4-97), for example the product Intelimer® IPA 13-1 from the company Landec, which is a polystearyl acrylate with a molecular weight of about 145 000 and a melting point of 49°C,
- homopolymers or copolymers bearing at least one crystallizable side chain, in particular containing fluoro group(s), as described in document WO-A-01/19333,
- acrylate/silicone copolymers, such as copolymers of acrylic acid and of stearyl acrylate bearing polydimethylsiloxane grafts, copolymers of stearyl methacrylate bearing polydimethylsiloxane grafts, copolymers of acrylic acid and of stearyl methacrylate bearing polydimethylsiloxane grafts, copolymers of methyl methacrylate, butyl methacrylate, 2-ethylhexyl acrylate and stearyl methacrylate bearing polydimethylsiloxane grafts. Mention may be made in particular of the copolymers sold by the company Shin-Etsu under the names KP-561 (CTFA name: acrylates/dimethicone), KP-541 (CTFA name: acrylates/dimethicone and isopropyl alcohol), KP-545 (CTFA name: acrylates/dimethicone and cyclopentasiloxane),
- and mixtures thereof.

Preferably, the amount of semi-crystalline polymer(s), preferably chosen from semi-crystalline polymers bearing crystallizable side chains, represents from 0.1% to 30% by weight of solids relative to the total weight of the oily phase, for example from 0.5% to 25% by weight, better still from 5% to 20% or even from 5% to 12% by weight, relative to the total weight of the oily phase.

## IV. Dextrin esters

The composition according to the present disclosure may comprise as lipophilic gelling agent at least one dextrin ester.

In particular, the composition preferably comprises at least one preferably $C_{12}$ to $C_{24}$ and in particular $C_{14}$ to $C_{18}$ fatty acid ester of dextrin, or mixtures thereof.

Preferably, the dextrin ester is an ester of dextrin and of a $C_{12}$-$C_{18}$ and in particular $C_{14}$-$C_{18}$ fatty acid.

Preferably, the dextrin ester is chosen from dextrin myristate and/or dextrin palmitate, and mixtures thereof.

According to a particular embodiment, the dextrin ester is dextrin myristate, such as the product sold especially under the name Rheopearl MKL-2 by the company Chiba Flour Milling.

According to a preferred embodiment, the dextrin ester is dextrin palmitate. This product may be chosen, for example, from those sold under the names Rheopearl TL®, Rheopearl KL® and Rheopearl® KL2 by the company Chiba Flour Milling.

In a particularly preferred manner, the oily phase of a composition according to the present disclosure may comprise from 0.1% to 30% by weight, preferably from 2% to 25% and preferably from 7.5% to 17% by weight of dextrin ester(s) relative to the total weight of the oily phase.

In a particularly preferred manner, the composition according to the present disclosure may comprise between

0.1% and 10% by weight and preferably between 0.5% and 5% by weight of dextrin palmitate relative to the total weight of the oily phase. The dextrin palmitate may especially be the product sold under the names Rheopearl TL®, Rheopearl KL® or Rheopearl® KL2 by the company Chiba Flour Milling.

**V. Polymers containing hydrogen bonding**

As representatives of polymers containing hydrogen bonding that are suitable for use in the present disclosure, mention may be made most particularly of polyamides and in particular hydrocarbon-based polyamides and silicone polyamides.

Polyamides

The oily phase of a composition according to the present disclosure may comprise at least one polyamide chosen from hydrocarbon-based polyamides and silicone polyamides, and mixtures thereof.

Preferably, the total content of polyamide(s) is between 0.1% and 30% by weight expressed as solids, preferably between 0.1% and 20% by weight and preferably between 0.5% and 10% by weight relative to the total weight of the oily phase.

For the purposes of the present disclosure, the term "*polyamide*" means a compound containing at least 2 repeating amide units, preferably at least 3 repeating amide units and better still 10 repeating amide units.

a) Hydrocarbon-based polyamide

The term "*hydrocarbon-based polyamide*" means a polyamide formed essentially of, indeed even consisting of, carbon and hydrogen atoms, and optionally of oxygen or nitrogen atoms, and not comprising any silicon or fluorine atoms. It may contain alcohol, ester, ether, carboxylic acid, amine and/or amide groups.

For the purposes of the present disclosure, the term "*functionalized chain*" means an alkyl chain comprising one or more functional groups or reagents chosen especially from hydroxyl, ether, ester, oxyalkylene and polyoxyalkylene groups.

Advantageously, this polyamide of the composition according to the present disclosure has a weight-average molecular mass of less than 100 000 g/mol (especially ranging from 1000 to 100 000 g/mol), in particular less than 50 000 g/mol (especially ranging from 1000 to 50 000 g/mol) and more particularly ranging from 1000 to 30 000 g/mol, preferably from 2000 to 20 000 g/mol and better still from 2000 to 10 000 g/mol.

This polyamide is insoluble in water, especially at 25°C.

According to a first embodiment of the present disclosure, the polyamide used is a polyamide of formula (I):

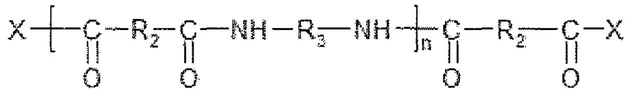

(I)

in which X represents a group -N($R_1$)$_2$ or a group -O$R_1$ in which $R_1$ is a linear or branched $C_8$ to $C_{22}$, alkyl radical which may be identical or different, $R_2$ is a $C_{28}$-$C_{42}$ diacid dimer residue, $R_3$ is an ethylenediamine radical and n is between 2 and 5;
and mixtures thereof.

According to a particular mode, the polyamide used is an amide-terminated polyamide of formula (Ia):

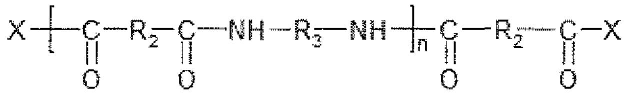

(Ia)

in which X represents a group -N($R_1$)$_2$ in which $R_1$ is a linear or branched $C_8$ to $C_{22}$, alkyl radical which may be identical or different, $R_2$ is a $C_{28}$-$C_{42}$ diacid dimer residue, $R_3$ is an ethylenediamine radical and n is between 2 and 5;
and mixtures thereof.

The oily phase of a composition according to the present disclosure may also comprise, additionally in this case, at least one additional polyamide of formula (Ib):

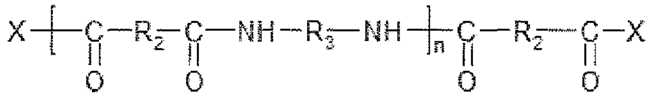

(Ib)

in which X represents a group -$OR_1$ in which $R_1$ is a linear or branched $C_8$ to $C_{22}$, and preferably $C_{16}$ to $C_{22}$ alkyl radical which may be identical or different, $R_2$ is a $C_{28}$-$C_{42}$ diacid dimer residue, $R_3$ is an ethylenediamine radical and n is between 2 and 5, such as the commercial products sold by the company Arizona Chemical under the names Uniclear 80 and Uniclear 100 or Uniclear 80 V, Uniclear 100 V and Uniclear 100 VG, the INCI name of which is Ethylenediamine/stearyl dimer dilinoleate copolymer.

b) Silicone polyamide

The silicone polyamides are preferably solid at room temperature (25°C) and atmospheric pressure (760 mmHg).

The silicone polyamides may preferentially be polymers comprising at least one unit of formula (III) or (IV):

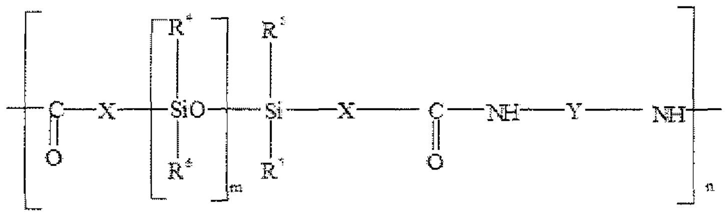

(III)

or

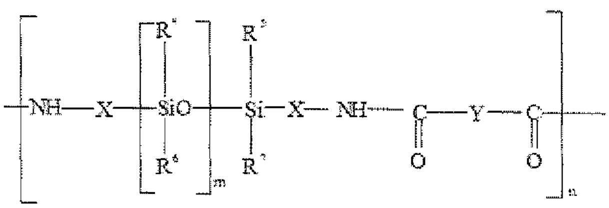

(IV)

in which:

- $R^4$, $R^5$, $R^6$ and $R^7$, which may be identical or different, represent a group chosen from:
  - saturated or unsaturated, $C_1$ to $C_{40}$ linear, branched or cyclic hydrocarbon-based groups, which may contain in their chain one or more oxygen, sulfur and/or nitrogen atoms, and which may be partially or totally substituted with fluorine atoms,
  - $C_6$ to $C_{10}$ aryl groups, optionally substituted with one or more $C_1$ to $C_4$ alkyl groups,
  - polyorganosiloxane chains possibly containing one or more oxygen, sulfur and/or nitrogen atoms,
- the groups X, which may be identical or different, represent a linear or branched $C_1$ to $C_{30}$ alkylenediyl group, possibly containing in its chain one or more oxygen and/or nitrogen atoms,
- Y is a saturated or unsaturated $C_1$ to $C_{50}$ linear or branched alkylene, arylene, cycloalkylene, alkylarylene or arylalkylene divalent group, which may comprise one or more oxygen, sulfur and/or nitrogen atoms, and/or may bear as substituent one of the following atoms or groups of atoms: fluorine, hydroxyl, $C_3$ to $C_8$ cycloalkyl, $C_1$ to $C_{40}$ alkyl, $C_5$ to $C_{10}$ aryl, phenyl optionally substituted with 1 to 3 $C_1$ to $C_3$ alkyl, $C_1$ to $C_3$ hydroxyalkyl and $C_1$ to $C_6$ aminoalkyl groups, or

Y represents a group corresponding to the formula:

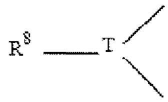

in which

- T represents a linear or branched, saturated or unsaturated, $C_3$ to $C_{24}$ trivalent or tetravalent hydrocarbon-based group optionally substituted with a polyorganosiloxane chain, and possibly containing one or more atoms chosen from O, N and S, or T represents a trivalent atom chosen from N, P and Al, and
- $R^8$ represents a linear or branched $C_1$ to $C_{50}$ alkyl group or a polyorganosiloxane chain, possibly comprising one or more ester, amide, urethane, thiocarbamate, urea, thiourea and/or sulfonamide groups, which may possibly be linked to another chain of the polymer,

  • n is an integer ranging from 2 to 500 and preferably from 2 to 200, and m is an integer ranging from 1 to 1000, preferably from 1 to 700 and even better still from 6 to 200.

According to a particular mode, the silicone polyamide comprises at least one unit of formula (III) in which m ranges from 50 to 200, in particular from 75 to 150 and is preferably about 100.
More preferably, $R^4$, $R^5$, $R^6$ and $R^7$ independently represent a linear or branched $C_1$ to $C_{40}$ alkyl group, preferably a group $CH_3$, $C_2H_5$, n-$C_3H_7$ or an isopropyl group in formula (III).
As an example of silicone polymers that may be used, mention may be made of one of the silicone polyamides obtained in accordance with Examples 1 to 3 of document US A 5 981 680.
Mention may be made of the compounds sold by the company Dow Corning under the names DC 2-8179 (DP 100) and DC 2-8178 (DP 15), the INCI name of which is Nylon-611/dimethicone copolymer, i.e. Nylon-611/dimethicone copolymers. The silicone polymers and/or copolymers advantageously have a temperature of transition from the solid state to the liquid state ranging from 45°C to 190°C. Preferably, they have a temperature of transition from the solid state to the liquid state ranging from 70 to 130°C and better still from 80°C to 105°C.
Preferably, the total content of polyamide(s) and/or silicone polyamide(s) is between 0.5% and 25% by weight of solids, in particular from 2% to 20% by weight and preferably between 2% and 12% by weight relative to the total weight of the oily phase.
Advantageously, the polymer containing hydrogen bonding is chosen from the ethylenediamine/stearyl dimer dilinoleate copolymer and Nylon-611/dimethicone copolymers.
A composition according to the invention comprises a lipophilic gelling agent chosen from organopolysiloxane elastomer.

## HYDROPHILIC GELLING AGENT/LIPOPHILIC GELLING AGENT SYSTEM

As preferred synthetic polymeric hydrophilic gelling agents, mention may be made more particularly of 2-acrylamido-2-methylpropanesulfonic acid copolymers and in particular copolymers of AMPS® and of hydroxyethyl acrylate, for instance the AMPS®/hydroxyethyl acrylate copolymer such as that used in the commercial product sold under the name Simulgel NS® b y the company SEPPIC (CTFA name: Hydroxyethyl acrylate/Sodium acryloyldimethyltaurate copolymer (and) Squalane (and) Polysorbate 60), or such as the product sold under the name Sodium acrylamido-2-methylpropanesulfonate/Hydroxyethyl acrylate copolymer, such as the commercial product Sepinov EMT 10 (INCI name: Hydroxyethyl acrylate/Sodium acryloyldimethyltaurate copolymer).
As preferred lipophilic gelling agent of organopolysiloxane elastomer type, mention may be made more particularly of crosslinked organopolysiloxane elastomers chosen from Dimethicone Crosspolymer (INCI name), Dimethicone (and) Dimethicone Crosspolymer (INCI name), Vinyl Dimethicone Crosspolymer (INCI name), Dimethicone/Vinyl Dimethicone Crosspolymer (INCI name), Dimethicone Crosspolymer-3 (INCI name), and in particular Dimethicone Crosspolymer (INCI name) and Dimethicone (and) Dimethicone Crosspolymer (INCI name).
According to a preferred mode, as preferred lipophilic gelling agents, mention may be made more particularly of gels of silicone elastomer dispersed in a silicone oil and/or powders of organopolysiloxane elastomer coated with silsesquioxane resin.
Thus, according to a particular mode, use is made of a gel of silicone elastomer dispersed in a silicone oil chosen from a non-exhaustive list comprising cyclopentadimethylsiloxane, dimethicones, dimethylsiloxanes, methyl trimethicone, phenyl methicone, phenyl dimethicone, phenyl trimethicone and cyclomethicone, preferably a linear silicone oil chosen from polydimethylsiloxanes (PDMS) or dimethicones with a viscosity at 25°C ranging from 1 to 500 cSt at 25°C, especially the following references:

- dimethicone/vinyl dimethicone crosspolymer (and) dimethicone, such as KSG-6 and KSG-16 from the company Shin-Etsu;
- dimethicone (and) dimethicone crosspolymer, such as DC9041 from the company Dow Corning; and
- dimethicone (and) dimethicone crosspolymer, such as Dow Corning EL-9240® Silicone Elastomer Blend from the company Dow Corning.

According to a particularly preferred embodiment, the composition according to the invention comprises at least one crosslinked silicone elastomer having the INCI name "dimethicone (and) dimethicone crosspolymer", with, preferably, a dimethicone having a viscosity ranging from 1 to 100 cSt, in particular from 1 to 10 cSt at 25°C, such as the mixture of polydimethylsiloxane with hexadiene/polydimethylsiloxane (5 cSt) sold under the name DC 9041 by Dow Corning and the mixture of polydimethylsiloxane with hexadiene/polydimethylsiloxane (2 cSt) sold under the name EL-9240® Silicone Elastomer Blend by Dow Corning.

According to another particularly preferred mode, the composition according to the invention comprises at least one powder of organopolysiloxane elastomer coated with silsesquioxane resin, of INCI name: vinyl dimethicone/methicone silsesquioxane crosspolymer, such as the reference KSP-100 sold by the company Shin-Etsu.

As non-limiting illustrations of hydrophilic gelling agent/lipophilic gelling agent systems that are most particularly suitable for use in the invention, mention may be made especially of the copolymer system of 2-acrylamido-2-methylpropanesulfonic acid/organopolysiloxane elastomer.

Thus, a composition according to the invention may advantageously comprise as hydrophilic gelling agent/lipophilic gelling agent system, a copolymer system of 2-acrylamido-2-methylpropanesulfonic acid and of hydroxyethyl acrylate/organopolysiloxane elastomer(s).

Preferably, a composition according to the invention may comprise as hydrophilic gelling agent/lipophilic gelling agent system, a copolymer system of 2-acrylamido-2-methylpropanesulfonic acid and of hydroxyethyl acrylate/organopolysiloxane elastomer powder.

## SOFT-FOCUS FILLERS

As stated previously, the claimed compositions comprise at least one soft-focus filler, chosen from acrylic copolymer powders, polymethyl methacrylate powders, talc/$TiO_2$ /alumina/silica composite powders, silica/$TiO_2$ composite powders, hollow hemispherical silicone particles, crosslinked elastomeric organopolysiloxane powders, crosslinked elastomeric organopolysiloxane powders coated with silicone resin, starch powders, polyamide powders, spherical cellulose beads, and mixtures thereof.

Fillers of this type are particularly advantageous insofar as they can make imperfections hazy. As indicated previously, the performance of these fillers is advantageously increased by means of using them in a composition according to the invention.

The soft-focus effect is characterized by Haze and transparency measurements (transmission TH). The Haze corresponds to the percentage of light scattered relative to the total transmittance according to standard ASTM D 1003 (Standard Test Method for Haze and Luminous Transmittance of Transparent Plastics).

25 $\mu$m films of composition are applied onto 50 $\mu$m polyethylene (PE) films. The film is then measured after 1 hour of drying at room temperature. Finally, the film is placed in the machine and transparency and Haze measurements are taken.

In particular, a composition according to the present disclosure comprises:

- at least one aqueous phase gelled with at least one synthetic polymeric hydrophilic gelling agent; at least one oily phase gelled with at least one lipophilic gelling agent, said phases forming therein a macroscopically homogeneous mixture;
- at least one soft-focus filler, and is characterized in that the Haze and the transmission TH are, respectively, greater than or equal to 75 according to standard ASTM D 1003 (Standard Test Method for Haze and Luminous Transmittance of Transparent Plastics).

Needless to say, as indicated below, the compositions according to the present disclosure may in parallel also contain standard additional fillers, it being understood that a person skilled in the art will take care to ensure that he does not choose fillers whose nature or amount in the composition would impair the soft-focus effect afforded by the soft-focus fillers.

For the purposes of the present disclosure, the term "*fillers*" should be understood as meaning colourless or white, mineral or organic, natural or synthetic solid particles of any form, which are in an insoluble and dispersed form in the medium of the composition.

The soft-focus fillers that may be used in the composition according to the present disclosure are especially

characterized by a refractive index of between 1.33 and 2.

They will generally comprise or consist of particles with a number-average size of less than or equal to 25 μm, especially less than or equal to 20 μm and in particular less than or equal to 15 μm.

The term "number-average size" denotes the dimension given by statistical particle size distribution for half the population, referred to as D50 measured with a Malvern Mastersizer machine.

These particles may be in any form and in particular may be spherical or non-spherical.

In particular, the soft-focus filler is chosen from polytetrafluoroethylene powders, polyurethane powders, carnauba microwaxes, synthetic wax microwaxes, silicone resin powders, hollow hemispherical silicone particles, acrylic copolymer powders, expanded vinylidene/acrylonitrile/methylene methacrylate microspheres, polyethylene powders, especially comprising at least one ethylene/acrylic acid copolymer, polymethyl methacrylate powders, crosslinked elastomeric organopolysiloxane powders, crosslinked elastomeric organopolysiloxane powders coated with silicone resin, starch powders, polyamide powders, powders of silica and silicates, especially of alumina, hydrophobic aerogel particles, talc having a number-average size of less than or equal to 3 microns, silica/$TiO_2$ composites, barium sulfate particles, boron nitride particles, silica particles surface-treated with a mineral wax at 1% to 2%, amorphous silica microspheres, silica microbeads, talc/$TiO_2$/alumina/silica composite powders, silicone elastomers, spherical cellulose beads, and mixtures thereof.

More particularly, the soft-focus filler is chosen from the group consisting of polytetrafluoroethylene powders, polyurethane powders, carnauba microwaxes, synthetic wax microwaxes, silicone resin powders, hollow hemispherical silicone particles, acrylic copolymer powders, expanded vinylidene/acrylonitrile/methylene methacrylate microspheres, polyethylene powders, especially comprising at least one ethylene/acrylic acid copolymer, polymethyl methacrylate powders, starch powders, polyamide powders, powders of silica and silicates, especially of alumina, hydrophobic aerogel particles, talc having a number-average size of less than or equal to 3 microns, silica/$TiO_2$ composites, barium sulfate particles, boron nitride particles, silica particles surface-treated with a mineral wax at 1% to 2%, amorphous silica microspheres, silica microbeads, talc/$TiO_2$/alumina/silica composite powders, spherical cellulose beads, and mixtures thereof.

According to a preferred mode, the composition comprises at least one soft-focus filler chosen from polymethyl methacrylate powders (bulk-filled or with a core-shell structure), talc/$TiO_2$/alumina/silica composite powders, silica/$TiO_2$ composite powders, hollow hemispherical silicone particles, crosslinked elastomeric organopolysiloxane powders, crosslinked elastomeric organopolysiloxane powders coated with silicone resin, starch powders, polyamide powders, spherical cellulose beads, and mixtures thereof.

According to another preferred mode, the composition comprises at least one soft-focus filler chosen from the group consisting of acrylic copolymer powders, polymethyl methacrylate powders (bulk-filled microspheres or microspheres with a core-shell structure), talc/$TiO_2$/alumina/silica composite powders, silica/$TiO_2$ composite powders, hollow hemispherical silicone particles, starch powders, polyamide powders, spherical cellulose beads, and mixtures thereof.

As soft-focus fillers that is used according to the invention, mention is made of:

- talc/$TiO_2$/alumina/silica composite powders, for instance thos e sold, for example, under the name Coverleaf AR-80® by the company Catalyst & Chemicals;
- silica/$TiO_2$ composites, such as those sold by the company Sunjin Chemical under the name Sunsil Tin 50;
- acrylic copolymer powders, especially of polymethyl (meth)acrylate, for instance the PMMA particles Jurimer MBI® from Nihon Junyoki with a mean size of 8 μm, the hollow PMMA spheres sold under the name Covabeads LH85® by the company Wackherr, the PMMA particles Ganzpearl GMP0820® from the company Ganz Chemical, and the expanded vinylidene/acrylonitrile/methylene methacrylate microspheres sold under the name Expancel®; hollow spheres of acrylate/ethylhexyl acrylate crosspolymer copolymer, such as the products sold by the company Daito Kasei Kogyo under the name Maquibeads SP-10; acrylate/ethylhexyl acrylate copolymer microspheres, such as those sold by the company Serisui Plastics under the name Techpolymer ACP-8C;
- hollow hemispherical silicone particles, of INCI name Methylsilanol/silicate crosspolymer, as described in patent applications JP-2003 128 788 and JP-A-2000-191789, for instance NLK 500®, NLK 506® and NLK 510® from Takemoto Oil and Fat;
- hydrophobic silica aerogel particles (INCI name: Silica silylate), such as the product sold by the company Dow Corning under the name Dow Corning VM-2270 Aerogel Fine Particles;
- crosslinked elastomeric organopolysiloxane powders, such as Dow Corning 9701 Cosmetic Powder® from the company Dow Corning (INCI name: Dimethicone/vinyl dimethicone crosspolymer); or the product sold by the company Dow Corning under the name EP-9801 Hydrocosmetic Powder (INCI name: Dimethicone/vinyl dimethicone crosspolymer (and) butylene glycol);
- crosslinked elastomeric organopolysiloxane powders coated with silicone resin, especially with silsesquioxane resin, as described, for example, in patent US 5 538 793. Such elastomers powders are sold under the names KSP-100®, KSP-101®, KSP-102®, KSP-103®, KSP-104® and KSP-105® by the company Shin-Etsu; or the product having the

INCI name Vinyl dimethicone/methicone silsesquioxane crosspolymer treated with PEG-7 glyceryl cocoate, Polyquaternium-7 and methylsilanol tri-PEG-8 glyceryl cocoate sold by the company Miyoshi Kasei under the name MW-SRP-100;
- starch powders, in particular aluminium starch octenylsuccinate, such as the product sold by the company Akzo Nobel under the name Dry Flo Plus;
- polyamide powders, such as Nylon® 12 powder, especially the product sold under the name Orgasol 2002 Extra D Nat Cos® by the company Atochem;
- spherical cellulose beads, such as those sold by the company Daito Kasei under the name Cellulobeads USF;
- and mixtures thereof.

According to a particular embodiment, the soft-focus fillers used according to the invention are chosen from:

- talc/$TiO_2$/alumina/silica composite powders, for instance thos e sold, for example, under the name Coverleaf AR-80® by the company Catalyst & Chemicals;
- acrylic copolymer powders, especially acrylate/ethylhexyl acrylate crosspolymer copolymer hollow spheres, such as the product sold by the company Daito Kasei Kogyo under the name Maquibeads SP-10;
- hollow hemispherical silicone particles, of INCI name Methylsilanol/silicate crosspolymer, as described in patent applications JP-2003 128 788 and JP-A-2000-191789, for instance NLK 506® from Takemoto Oil and Fat;
- crosslinked elastomeric organopolysiloxane powders, such as the product sold by the company Dow Corning under the name EP-9801 Hydrocosmetic Powder (INCI name: Dimethicone/vinyl dimethicone crosspolymer (and) butylene glycol);
- crosslinked elastomeric organopolysiloxane powders coated with silicone resin, especially with silsesquioxane resin, such as the products sold under the names KSP-100®, KSP-101®, KSP-102®, KSP-103®, KSP-104® and KSP-105® by the company Shin-Etsu; or the product having the INCI name Vinyl dimethicone/methicone silsesquioxane crosspolymer treated with PEG-7 glyceryl cocoate, Polyquaternium-7 and methylsilanol tri-PEG-8 glyceryl cocoate sold by the company Miyoshi Kasei under the name MW-SRP-100;
- and mixtures thereof.

Even more preferably, the soft-focus fillers used according to the invention are chosen from:

- talc/$TiO_2$/alumina/silica composite powders, for instance thos e sold, for example, under the name Coverleaf AR-80® by the company Catalyst & Chemicals;
- crosslinked elastomeric organopolysiloxane powders, such as the product sold by the company Dow Corning under the name EP-9801 Hydrocosmetic Powder (INCI name: Dimethicone/vinyl dimethicone crosspolymer (and) butylene glycol);
- crosslinked elastomeric organopolysiloxane powders coated with silicone resin, especially with silsesquioxane resin, such as the products sold under the names KSP-100®, KSP-101®, KSP-102®, KSP-103®, KSP-104® and KSP-105® by the company Shin-Etsu; or the product having the INCI name Vinyl dimethicone/methicone silsesquioxane crosspolymer treated with PEG-7 glyceryl cocoate, Polyquaternium-7 and methylsilanol tri-PEG-8 glyceryl cocoate sold by the company Miyoshi Kasei under the name MW-SRP-100;
- and mixtures thereof.

According to one embodiment, said soft-focus filler(s) are present totally or partly, and preferably solely, in the gelled aqueous phase.
According to another embodiment, said soft-focus filler(s) are present totally or partly, and preferably solely, in the gelled oily phase.
According to another embodiment, the composition comprises a soft-focus filler in the gelled aqueous phase and a second soft-focus filler in the oily phase, the latter filler also possibly acting as oily gelling agent.
In particular, when the soft-focus filler also acts as oily gelling agent, then it is present in a content of greater than 12% by weight and preferably greater than 15% by weight relative to the total weight of the composition.
According to another embodiment, in a composition according to the invention, the soft-focus filler(s) are different from the oily gelling agent(s).
A composition according to the invention may comprise from 0.2% to 40% by weight, especially from 0.5% to 37% by weight, in particular from 0.75% to 35% by weight and preferably from 1% to 30% by weight of soft-focus filler(s) relative to the total weight of said composition.

## AQUEOUS PHASE

The aqueous phase of a composition according to the invention comprises water and optionally a water-soluble solvent.

In the present invention, the term "water-soluble solvent" denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition in accordance with the invention, mention may be made especially of lower monoalcohols containing from 1 to 5 carbon atoms such as ethanol and isopropanol, glycols containing from 2 to 8 carbon atoms such as ethylene glycol, propylene glycol, 1,3-butylene glycol and dipropylene glycol, $C_3$ and $C_4$ ketones and $C_2$-$C_4$ aldehydes.

The aqueous phase (water and optionally the water-miscible solvent) may be present in the composition in a content ranging from 5% to 95%, better still from 30% to 80% by weight and preferably from 40% to 75% by weight relative to the total weight of said composition.

According to another embodiment variant, the aqueous phase of a composition according to the invention may comprise at least one $C_2$-$C_{32}$ polyol.

For the purposes of the present invention, the term "*polyol*" should be understood as meaning any organic molecule comprising at least two free hydroxyl groups.

Preferably, a polyol in accordance with the present invention is present in liquid form at room temperature.

A polyol suitable for the invention can be a compound of saturated or unsaturated and linear, branched or cyclic alkyl type carrying, on the alkyl chain, at least two -OH functional groups, in particular at least three -OH functional groups and more particularly at least four -OH functional groups.

The polyols advantageously suitable for the formulation of a composition according to the present invention are those exhibiting in particular from 2 to 32 carbon atoms and preferably from 3 to 16 carbon atoms.

Advantageously, the polyol may be chosen, for example, from ethylene glycol, pentaerythritol, trimethylolpropane, propylene glycol, 1,3 -propanediol, butylene glycol, isoprene glycol, pentylene glycol, hexylene glycol, glycerol, polyglycerols such as glycerol oligomers, for instance diglycerol, and polyethylene glycols, and mixtures thereof.

According to a preferred embodiment of the invention, said polyol is chosen from ethylene glycol, pentaerythritol, trimethylolpropane, propylene glycol, glycerol, polyglycerols, polyethylene glycols and mixtures thereof.

According to a particular mode, the composition of the invention may comprise at least propylene glycol.

According to another particular mode, the composition of the invention may comprise at least glycerol.

## OILY PHASE

For the purposes of the invention, an oily phase comprises at least one oil.

The term "*oil*" means any fatty substance that is in liquid form at room temperature and atmospheric pressure.

An oily phase that is suitable for preparing the cosmetic compositions according to the invention may comprise hydrocarbon-based oils, silicone oils, fluoro oils or non-fluoro oils, or mixtures thereof.

The oils may be volatile or non-volatile.

They may be of animal, plant, mineral or synthetic origin. According to one embodiment variant, oils of plant origin are preferred.

For the purposes of the present invention, the term "non-volatile oil" means an oil with a vapour pressure of less than 0.13 Pa.

For the purposes of the present invention, the term "silicone oil" means an oil comprising at least one silicon atom, and in particular at least one Si-O group.

The term "*fluoro oil*" means an oil comprising at least one fluorine atom.

The term "*hydrocarbon-based oil*" means an oil mainly containing hydrogen and carbon atoms.

The oils may optionally comprise oxygen, nitrogen, sulfur and/or phosphorus atoms, for example in the form of hydroxyl or acid radicals.

For the purposes of the invention, the term "*volatile oil*" means any oil that is capable of evaporating on contact with the skin in less than one hour, at room temperature and atmospheric pressure. The volatile oil is a volatile cosmetic compound, which is liquid at room temperature, especially having a nonzero vapour pressure, at room temperature and atmospheric pressure, in particular having a vapour pressure ranging from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

## Volatile oils

The volatile oils may be hydrocarbon-based oils or silicone oils.

Among the volatile hydrocarbon-based oils containing from 8 to 16 carbon atoms, mention may be made especially of branched $C_8$-$C_{16}$ alkanes, such as $C_8$-$C_{16}$ isoalkanes (also known as isoparaffins), isododecane, isodecane, isohexadecane and, for example, the oils sold under the trade names Isopar or Permetyl, branched $C_8$-$C_{16}$ esters, such as isohexyl neopentanoate, and mixtures thereof. Preferably, the volatile hydrocarbon-based oil is chosen from volatile hydrocarbon-based oils containing from 8 to 16 carbon atoms, and mixtures thereof, in particular from isododecane, isodecane isohexadecane, and is especially isohexadecane.

Mention may also be made of volatile linear alkanes comprising from 8 to 16 carbon atoms, in particular from 10 to 15 carbon atoms and more particularly from 11 to 13 carbon atoms, for instance n-dodecane ($C_{12}$) and n-tetradecane ($C_{14}$) sold by Sasol under the respective references Parafol 12-97 and Parafol 14-97, and also mixtures thereof, the undecane-tridecane mixture, mixtures of n-undecane ($C_{11}$) and of n-tridecane ($C_{13}$) obtained in Examples 1 and 2 of patent application WO 2008/155 059 from the company Cognis, and mixtures thereof.

Volatile silicone oils that may be mentioned include linear volatile silicone oils such as hexamethyldisiloxane, octamethyltrisiloxane, decamethyltetrasiloxane, tetradecamethylhexasiloxane, hexadecamethylheptasiloxane and dodecamethylpentasiloxane.

Volatile cyclic silicone oils that may be mentioned include hexamethylcyclotrisiloxane, octamethylcyclotetrasiloxane, decamethylcyclopentasiloxane and dodecamethylcyclohexasiloxane.

## Non-volatile oils

The non-volatile oils may be chosen especially from non-volatile hydrocarbon-based, fluoro and/or silicone oils.

Non-volatile hydrocarbon-based oils that may especially be mentioned include:

- hydrocarbon-based oils of animal origin,
- hydrocarbon-based oils of plant origin, synthetic ethers containing from 10 to 40 carbon atoms, such as dicaprylyl ether,
- synthetic esters, such as the oils of formula $R_1COOR_2$, in which $R_1$ represents a linear or branched fatty acid residue comprising from 1 to 40 carbon atoms and $R_2$ represents a hydrocarbon-based chain, which is especially branched, containing from 1 to 40 carbon atoms, on condition that $R_1 + R_2 \geq 10$. The esters may be chosen especially from fatty acid alcohol esters, for instance cetostearyl octanoate, isopropyl alcohol esters such as isopropyl myristate or isopropyl palmitate, ethyl palmitate, 2-ethylhexyl palmitate, isopropyl stearate, octyl stearate, hydroxylated esters, such as isostearyl lactate or octyl hydroxystearate, alkyl or polyalkyl ricinoleates, hexyl laurate, neopentanoic acid esters, such as isodecyl neopentanoate or isotridecyl neopentanoate, and isononanoic acid esters, such as isononyl isononanoate or isotridecyl isononanoate,
- polyol esters and pentaerythritol esters, such as dipentaerythritol tetrahydroxystearate/tetraisostearate,
- fatty alcohols that are liquid at room temperature, with a branched and/or unsaturated carbon-based chain containing from 12 to 26 carbon atoms, such as 2-octyldodecanol, isostearyl alcohol and oleyl alcohol,
- $C_{12}$-$C_{22}$ higher fatty acids, such as oleic acid, linoleic acid, linolenic acid, and mixtures thereof,
- non-phenyl silicone oils, for instance caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, dimethicones or phenyl trimethicone with a viscosity of less than or equal to 100 cSt, and trimethylpentaphenyltrisiloxane, and mixtures thereof; and also mixtures of these various oils.

Preferably, a composition according to the invention comprises volatile and/or non-volatile silicone oils.

A composition according to the invention may comprise from 5% to 95% by weight, better still from 5% to 40% by weight and preferably from 7% to 35% by weight of oil(s) relative to the total weight of said composition.

As mentioned above, the gelled oily phase according to the invention may have a threshold stress of greater than 1.5 Pa and preferably greater than 10 Pa. This threshold stress value reflects a gel-type texture of this oily phase.

## DYESTUFFS

A composition according to the invention may also comprise at least one particulate or non-particulate, water-soluble or water-insoluble dyestuff, preferably in a proportion of at least 0.01% by weight relative to the total weight of the composition.

For obvious reasons, this amount is liable to vary significantly with regard to the intensity of the desired colour

effect and of the colour intensity afforded by the dyestuffs under consideration, and its adjustment clearly falls within the competence of a person skilled in the art.

A composition according to the invention may comprise from 0.01% to 25% by weight, especially from 0.1% to 25% by weight, in particular from 1% to 20% by weight and preferably from 2.5% to 15% by weight of dyestuffs relative to the total weight of said composition.

As stated above, the dyestuffs that are suitable for use in the invention may be water-soluble, but may also be liposoluble.

For the purposes of the invention, the term "*water-soluble dyestuff*" means any natural or synthetic, generally organic compound, which is soluble in an aqueous phase or water-miscible solvents and which is capable of imparting colour.

As water-soluble dyes that are suitable for use in the invention, mention may be made especially of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanine (beetroot), carmine, copper chlorophylline, methylene blue, anthocyanins (enocianin, black carrot, hibiscus and elder), caramel and riboflavin.

The water-soluble dyes are, for example, beetroot juice and caramel.

For the purposes of the invention, the term "*liposoluble dyestuff*" means any natural or synthetic, generally organic compound, which is soluble in an oily phase or in solvents that are miscible with a fatty substance, and which is capable of imparting colour.

As liposoluble dyes that are suitable for use in the invention, mention may be made especially of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.

These colouring particulate materials may be present in a proportion of from 0.01% to 15% by weight relative to the total weight of the composition containing them.

They may especially be pigments, nacres and/or particles with metallic tints.

The term "*pigments*" should be understood as meaning white or coloured, mineral or organic particles that are insoluble in an aqueous solution, which are intended to colour and/or opacify the composition containing them.

A composition according to the invention may comprise from 0.01% to 25% by weight, especially from 0.1% to 25% by weight, in particular from 1% to 25% by weight and preferably from 2.5% to 15% by weight of pigments relative to the total weight of said composition.

Preferably, when the composition according to the invention is a makeup composition, it may comprise at least 2.5% and preferentially at least 10% by weight of pigments, relative to the total weight of said composition.

The pigments may be white or coloured, and mineral and/or organic.

As mineral pigments that may be used in the invention, mention may be made of titanium oxide, titanium dioxide, zirconium oxide, zirconium dioxide, cerium oxide or cerium dioxide and also zinc oxide, iron oxide or chromium oxide, ferric blue, manganese violet, ultramarine blue and chromium hydrate, and mixtures thereof.

It may also be a pigment having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

They may also be pigments having a structure that may be, for example, of silica microsphere type containing iron oxide. An example of a pigment having this structure is the product sold by the company Miyoshi under the reference PC Ball PC-LL-100 P, this pigment consisting of silica microspheres containing yellow iron oxide.

Advantageously, the pigments in accordance with the invention are iron oxides and/or titanium dioxides.

The term "*nacres*" should be understood as meaning iridescent or non-iridescent coloured particles of any shape, especially produced by certain molluscs in their shell or alternatively synthesized, which have a colour effect via optical interference.

A composition according to the invention may comprise from 0% to 15% by weight of nacres relative to the total weight of said composition.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic dyestuffs.

Examples of nacres that may also be mentioned include natural mica coated with titanium oxide, with iron oxide, with natural pigment or with bismuth oxychloride.

Among the commercially available nacres that may be mentioned are the nacres Timica, Flamenco and Duochrome (on mica base) sold by the company Engelhard, the Timiron nacres sold by the company Merck, the Prestige nacres on mica base sold by the company Eckart and the Sunshine nacres on synthetic mica base sold by the company Sun Chemical.

The nacres may more particularly have a yellow, pink, red, bronze, orange, brown, gold and/or coppery colour or tint.

Advantageously, the nacres in accordance with the invention are micas coated with titanium dioxide or with iron oxide, and also bismuth oxychloride.

For the purposes of the present invention, the term "*particles with a metallic tint*" means any compound whose nature, size, structure and surface finish allow it to reflect the incident light, especially in a non-iridescent manner.

The particles with a metallic tint that may be used in the invention are in particular chosen from:

- particles of at least one metal and/or of at least one metal derivative,
- particles comprising a monomaterial or multimaterial, organic or mineral substrate, at least partially coated with at least one layer with a metallic tint comprising at least one metal and/or at least one metal derivative, and
- mixtures of said particles.

Among the metals that may be present in said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo and Cr, and mixtures or alloys thereof (for example bronzes and brasses) are preferred metals.

The term "*metal derivatives*" denotes compounds derived from metals, especially oxides, fluorides, chlorides and sulfides.

Illustrations of these particles that may be mentioned include aluminium particles, such as those sold under the names Starbrite 1200 EAC® by the company Siberline and Metalure® by the company Eckart and glass particles coated with a metallic layer, especially those described in documents JP-A-09188830, JP-A-10158450, JP-A-10158541, JP-A-07258460 and JP-A-05017710.

Hydrophobic treatment of the dyestuffs

The pulverulent dyestuffs as described previously may be totally or partially surface-treated, with a hydrophobic agent, to make them more compatible with the oily phase of the composition of the invention, especially so that they have good wettability with oils. Thus, these treated pigments are well dispersed in the oily phase.

Hydrophobic-treated pigments are described especially in document EP-A-1 086 683.

The hydrophobic-treatment agent may be chosen from silicones such as methicones, dimethicones and perfluoroalkylsilanes; fatty acids, such as stearic acid; metal soaps, such as aluminium dimyristate, the aluminium salt of hydrogenated tallow glutamate; perfluoroalkyl phosphates, polyhexafluoropropylene oxides; perfluoropolyethers; amino acids; N-acylamino acids or salts thereof; lecithin, isopropyl triisostearyl titanate, isostearyl sebacate, and mixtures thereof.

The term "alkyl" mentioned in the compounds cited above especially denotes an alkyl group containing from 1 to 30 carbon atoms and preferably containing from 5 to 16 carbon atoms.

## ADDITIONAL FILLERS

Advantageously, a composition according to the invention may comprise, besides one or more soft-focus fillers, one or more fillers conventionally used in care and/or makeup compositions.

These additional fillers are colourless or white solid particles of any form, which are in a form that is insoluble and dispersed in the medium of the composition.

These fillers, of mineral or organic, natural or synthetic nature, give the composition containing them softness and give the makeup result a matt effect and uniformity.

In particular, such additional fillers may be present in a composition according to the invention in a content of between 0.5% and 10% by weight, especially between 0.5% and 7% by weight and in particular between 0.5% and 5% by weight relative to the total weight of the composition.

According to one embodiment of the invention, a composition may comprise at least solid particles such as pigments and/or additional fillers.

Advantageously, a composition according to the invention may comprise from 0.01% to 25% by weight, especially from 0.1% to 25% by weight, in particular from 1% to 20% by weight and preferably from 5% to 15% by weight of solid particles relative to the total weight of the composition.

## DISPERSANT

Advantageously, a composition according to the invention may also comprise a dispersant.

Such a dispersant may be a surfactant, an oligomer, a polymer or a mixture of several thereof.

According to one particular embodiment, a dispersant in accordance with the invention is a surfactant.
According to a particular embodiment variant, a composition according to the invention comprises less than 1% by weight of surfactant relative to the total weight of the composition, or even is free of surfactant.

## ACTIVE AGENT

For a particular care application, a composition according to the invention may comprise at least one moisturizer (also known as a humectant).
Preferably, the moisturizer is glycerol.
The moisturizer(s) may be present in the composition in a content ranging from 0.1% to 15% by weight, especially from 0.5% to 10% by weight or even from 1% to 6% by weight relative to the total weight of said composition.
As other active agents that may be used in the composition of the invention, examples that may be mentioned include vitamins and sunscreens, and mixtures thereof.
Preferably, a composition according to the invention comprises at least one active agent.
It is a matter of routine operations for a person skilled in the art to adjust the nature and the amount of the additives present in the compositions in accordance with the invention such that the desired cosmetic properties thereof are not thereby affected.
According to one embodiment, a composition of the invention may advantageously be in the form of a composition for caring for the skin of the body or the face, in particular the face.
According to another embodiment, a composition of the invention may advantageously be in the form of a makeup base composition.
According to another embodiment, a composition of the invention may advantageously be in the form of a foundation.
According to one embodiment, a composition of the invention may advantageously be in the form of a composition for making up the skin and especially the face. It may thus be an eyeshadow or a face powder.
According to another embodiment, a composition of the invention may advantageously be in the form of a lip product, in particular a lipstick.
According to another embodiment, a composition of the invention may be in the form of a product for the eyelashes, in particular a mascara.
Such compositions are especially prepared according to the general knowledge of a person skilled in the art.
Throughout the description, including the claims, the term "*comprising a*" should be understood as being synonymous with *"comprising at least one",* unless otherwise specified.
The terms *"between... and..."* and *"ranging from... to..."* should be understood as being inclusive of the limits, unless otherwise specified.
The invention is illustrated in greater detail by the examples and figures presented below. Unless otherwise mentioned, the amounts indicated are expressed as mass percentages.

## METHODOLOGY FOR THE OSCILLATING DYNAMIC RHEOLOGY MEASUREMENTS

These are harmonic-regime rheology measurements for measuring the elastic modulus.
The measurements are taken using a Haake RS600 rheometer on a product at rest, at 25°C with a plate-plate rotor ∅ 60 mm and a 2 mm gap.
The harmonic-regime measurements make it possible to characterize the viscoelastic properties of the products. The technique consists in subjecting a material to a stress which varies sinusoidally over time and in measuring the response of the material to this stress. In a range in which the behaviour is linear viscoelastic behaviour (zone in which the strain is proportional to the stress), the stress ($\tau$) and the strain ($\gamma$) are two sinusoidal functions of time which are written in the following manner:

$$\tau(t) = \tau_0 \sin(\omega t)$$

$$\gamma(t) = \gamma_0 \sin(\omega t + \delta)$$

in which:

$\tau_0$ represents the maximum amplitude of the stress (Pa);
$\gamma_0$ represents the maximum amplitude of the strain (-);

$\omega = 2\Pi N$ represents the angular frequency ($rad.s^{-1}$) with N representing the frequency (Hz); and
$\delta$ represents the phase shift of the stress relative to the strain (rad).

Thus, the two functions have the same angular frequency, but they are shifted by an angle $\delta$. Depending on the phase shift $\delta$ between $\tau(t)$ and $\gamma(t)$, the behaviour of the system may be apprehended:

- if $\delta = 0$, the material is purely elastic;
- if $\delta = \Pi/2$, the material is purely viscous (Newtonian fluid); and
- if $0 < \delta < \Pi/2$, the material is viscoelastic.

In general, the stress and the strain are written in complex form:

$$\tau^*(t) = \tau_0\, e^{i\omega t}$$

$$\gamma^*(t) = \gamma_0\, e^{(i\omega t + \delta)}$$

A complex stiffness modulus, representing the overall resistance of the material to the strain, whether it is of elastic or viscous origin, is then defined by:

$$G^* = \tau^* / \gamma^* = G' + iG''$$

in which:

G' is the storage modulus or elastic modulus, which characterizes the energy stored and totally restituted during a cycle, $G' = (\tau_0 / \gamma_0) \cos \delta$; and
G" is the loss modulus or viscous modulus, which characterizes the energy dissipated by internal friction during a cycle, $G'' = (\tau_0 / \gamma_0) \sin \delta$.

The parameter retained is the mean stiffness modulus G* recorded at the plateau measured at a frequency of 1 Hz.

## EXAMPLES

### Example 1: Smoothing care composition according to the invention

A smoothing care formulation in accordance with the invention is prepared as described below.
The components of phase A are weighed out in a beaker and stirred with a Rayneri blender, at room temperature.
The components of phase Al are weighed out and then added to phase A at room temperature, and the mixture is then left to homogenize, by stirring with a Rayneri blender for 5 minutes, during which the walls of the beaker are scraped with a spatula.
After homogenization of the gel, the components of phases B and B1, which have been weighed out, are gradually added at room temperature until fully incorporated over 5 to 10 minutes, with vigorous stirring.
The walls are scraped with a spatula and the mixture is left to homogenize for 5 minutes with vigorous stirring using a Rayneri blender, at room temperature.
The formulation is prepared using the weight proportions described below.

| Phase | Compounds | Formulation (weight % relative to the total weight of the composition) |
|---|---|---|
| *Phase A* | Water | qs 300 |
| | Glycerol | 10.00 |
| | Preserving agents | 0.70 |
| | Hydroxyethyl acrylate/sodium acryloyldimethyltaurate copolymer (Sepinov® EMT 10 sold by the company SEPPIC) | 0.33 |
| *Phase A1* | Dimethicone/vinyl dimethicone crosspolymer (and) butylene glycol sold by the company Dow Corning under the name EP-9801 Hydrocosmetic Powder | 12.00 |
| *Phase B* | Dimethicone | 8.00 |
| *Phase B1* | Vinyl dimethicone/methicone silsesquioxane crosspolymer sold by the company Shin-Etsu under the name KSP-100 | 16.00 |

The composition according to the invention, when applied, shows very good soft-focus qualities. In particular, a marked decrease in pores is observed.

**Example 2: Gel-gel compositions according to the invention**

In order to demonstrate the soft-focus properties of the gel-gel compositions according to the invention, measurements were taken.

1) Preparation of thegel-gel compositions

Formulations in accordance with the invention are prepared as described below.
The components of phase A are weighed out in a beaker and stirred with a Rayneri blender, at room temperature.
After homogenization of the gel, the components of phase B, which have been weighed out, are gradually added at room temperature, with vigorous stirring.
The gel-gel composition forms.
The mixture is left to homogenize with stirring using a Rayneri blender for about 5 minutes at room temperature, while scraping the walls of the beaker with a spatula.
The mixture is stirred vigorously with a Rayneri blender for about 5 minutes, at room temperature.
The formulation is prepared using the weight proportions described below.

| Phase | Compounds | Formulation (weight % relative to the total weight of the composition) |
|---|---|---|
| *Phase A* | Water | qs 100 |
| | Glycerol | 6.60 |
| | Phenoxyethanol | 0.98 |
| | Hydroxyethyl acrylate/sodium acryloyldimethyltaurate copolymer (Sepinov® EMT 10 sold by the company SEPPIC) | 3 |
| *Phase B* | Dimethicone | 10.00 |
| | Dimethicone 84.5%/dimethicone crosspolymer 15.5% (DC9041® sold by the company Dow Corning) (*% of dimethicone crosspolymer solids) | 17.50 (2,71*) |

2) Addition of the soft-focus fillers

Five gel-gel compositions as described above are prepared and in each of them a soft-focus filler is added in an amount equal to 4% by weight relative to the total weight of the test composition.
Each of the following soft-focus fillers is tested:

- dimethicone/vinyl dimethicone crosspolymer (and) butylene glycol sold by the company Dow Corning under the name EP-9801 Hydrocosmetic Powder;
- methyl methacrylate crosspolymer, sold by the company Daito Kasei Kogyo under the name Maquibeads SP-10;
- talc (and) silica (and) alumina (and) titanium dioxide sold by the company Catalysts & Chemicals under the name Coverleaf AR-80;
- vinyl dimethicone/methicone silsesquioxane crosspolymer treated with PEG-7 glyceryl cocoate, Polyquaternium-7 and methylsilanol tri-PEG-8 glyceryl cocoate sold by the company Miyoshi Kasei under the name MW-SRP-100; and
- methysilanol/silicate crosspolymer sold by the company Takemoto Oil & Fat under the name NLK 506.

3) Evaluation of the soft-focus effect

The various formulations were spread to a thickness of 25 microns on a 50 μm polyethylene film and the soft-focus effect of each composition was evaluated with various methods for measuring the total Transmittance and the Haze.
The Haze corresponds to the percentage of light scattered relative to the total transmittance according to standard ASTM D 1003 (Standard Test Method for Haze and Luminous Transmittance of Transparent Plastics).
The results are given in the table below:

| Fillers | Gel-gel composition | |
|---|---|---|
| | Transmittance | Haze |
| EP-9801 Hydrocosmetic Powder | 93.5 | 78.2 |
| Maquibeads SP-10 | 93.2 | 68.8 |
| Coverleaf AR-80 | 92.6 | 68.2 |
| MW-SRP-100 | 93.3 | 68.1 |
| NLK 506 | 91.4 | 57.8 |

A high Haze reflects a substantial soft-focus effect.
The gel-gel compositions incorporating the soft-focus fillers have high Haze values. Thus, it is proven that the gel-gel compositions according to the invention make it possible to obtain a substantial soft-focus effect.

**Example 3: Comparison of the effect of the gel-gel compositions relative to aqueous or oily mono-gel compositions**

The "booster" effect of the gel-gel compositions on the properties of the soft-focus fillers, compared with mono-gel compositions, was demonstrated by taking soft-focus measurements.
Thus, the properties of bi-gel compositions comprising different soft-focus fillers were compared with those of mono-gel compositions, containing the same fillers at the same concentration.

1) Preparation of the gel-gel composition

The gel-gel composition to which the fillers were added is the same as that of Example 2.

2) Preparation of the aqueous mono-gel composition

The components of phase A are weighed out in a beaker and stirred with a Rayneri blender, at room temperature.

The component of phase B is weighed out in a capsule and added with vigorous stirring using a Rayneri blender to phase A, at room temperature.
The product thickens and the aqueous gel forms.
The mixture is stirred for about 5 minutes at room temperature, while scraping the walls of the beaker with a spatula, until fully homogenized.
The formulation is prepared using the weight proportions described below.

| Phase | Compounds | Formulation (weight % relative to the total weight of the composition) |
|---|---|---|
| A | Water | qs 100 |
| | Glycerol | 9.10 |
| | Preserving agents | 1.35 |
| B | Hydroxyethyl acrylate/sodium acryloyldimethyltaurate copolymer (Sepinov® EMT 10 sold by the company SEPPIC) | 4.15 |

3) Preparation of the oily mono-gel composition

The components of phase A are weighed out in a beaker and stirred vigorously with a Rayneri blender, at room temperature.
The gel forms.
The mixture is stirred for about two times 5 minutes at room temperature, while scraping the walls of the beaker with a spatula, until fully homogenized.
The formulation is prepared using the weight proportions described below.

| Phase | Compounds | Formulation (weight % relative to the total weight of the composition) |
|---|---|---|
| A | Dimethicone | 36.36 |
| | Dimethicone 84.5%/dimethicone crosspolymer 15.5% (DC9041® sold by the company Dow Coming) (*% of dimethicone crosspolymer solids) | 64.64 |

4) Addition of the soft-focus fillers

A soft-focus filler is added to each of the gel-gel and mono-gel compositions described above in points 1) to 3), in an amount equal to 4% by weight relative to the total weight of the test composition.
Each of the following soft-focus fillers is tested in each of the aqueous gel-gel and mono-gel compositions:

- dimethicone/vinyl dimethicone crosspolymer (and) butylene glycol sold by the company Dow Corning under the name EP-9801 Hydrocosmetic Powder;
- talc (and) silica (and) alumina (and) titanium dioxide sold by the company Catalysts & Chemicals under the name Coverleaf AR-80; and
- vinyl dimethicone/methicone silsesquioxane crosspolymer treated with PEG-7 glyceryl cocoate, Polyquaternium-7 and methylsilanol tri-PEG-8 glyceryl cocoate sold by the company Miyoshi Kasei under the name MW-SRP-100.

The filler Methylsilanol/silicate crosspolymer sold by the company Takemoto Oil & Fat under the name NLK 506 was tested in the gel-gel composition and in the oily mono-gel composition.

5)Evaluation of the soft-focus effect

The various formulations were spread to a thickness of 25 microns on a 50 μm polyethylene film and the soft-focus effect of each composition was evaluated with various methods for measuring the total Transmittance and the Haze, as indicated in Example 2.
The results are given in the table below:

| Fillers | Bi-gel composition | | Aqueous mono-gel composition | | Oily mono-gel composition | |
|---|---|---|---|---|---|---|
| | Transmittance | Haze | Transmittance | Haze | Transmittance | Haze |
| EP-9801 Hydrocosmetic Powder | 93.5 | 78.2 | 93.4 | 41.2 | / | / |
| MW-SRP-100 | 93.3 | 68.1 | 93.0 | 54.0 | / | / |
| NLK 506 | 91.4 | 57.8 | / | / | 93.0 | 44.7 |
| Coverleaf AR-80 | 92.6 | 68.2 | 93.1 | 55.0 | / | / |
| */: not measurable since the nature of the filler (lipophilic or hydrophilic) is incompatible with the medium into which it is introduced.* | | | | | | |

As indicated previously, a high Haze value reflects a substantial soft-focus effect.

The gel-gel compositions incorporating the soft-focus fillers have the highest Haze values in comparison with the mono-gel compositions comprising the same fillers in the same content.

The gel-gel compositions thus have a "booster" effect on the properties of the soft-focus fillers, when compared with the aqueous or oily mono-gel compositions.

## Claims

1. Cosmetic composition, different from an emulsion, for making up and/or caring for keratin materials, in particular the skin and/or the lips, comprising:

   - at least one aqueous phase gelled with at least one synthetic polymeric hydrophilic gelling agent chosen from crosslinked and/or neutralized 2-acrylamido-2-methylpropanesulfonic acid copolymers; and
   - at least one oily phase gelled with at least one lipophilic gelling agent chosen from organopolysiloxane elastomers;

   said phases forming therein a macroscopically homogeneous mixture;
   said composition also comprising at least one soft-focus filler chosen from acrylic copolymer powders, polymethyl methacrylate powders, talc/$TiO_2$/alumina/silica composite powders, silica/$TiO_2$ composite powders, hollow hemispherical silicone particles, crosslinked elastomeric organopolysiloxane powders, crosslinked elastomeric organopolysiloxane powders coated with silicone resin, starch powders, polyamide powders, spherical cellulose beads, and mixtures thereof.

2. Composition according to the preceding claim, comprising from 0.2% to 40% by weight, especially from 0.5% to 37% by weight, in particular from 0.75% to 35% by weight and preferably from 1% to 30% by weight of soft-focus filler(s) relative to the total weight of said composition.

3. Composition according to either of the preceding claims, in which said soft-focus filler(s) are totally or partly, and preferably solely, present in the gelled aqueous phase or are totally or partly, and preferably solely, present in the gelled oily phase.

4. Composition according to either of the preceding claims, comprising as synthetic polymeric hydrophilic gelling agent at least one copolymer of 2-acrylamido-2-methylpropanesulfonic acid and of hydroxy ethyl acrylate.

5. Composition according to any one of the preceding claims, in which said lipophilic gelling agent is chosen from Dimethicone Crosspolymer, Dimethicone (and) Dimethicone Crosspolymer, Vinyl Dimethicone Crosspolymer, Dimethicone/Vinyl Dimethicone Crosspolymer, Dimethicone Crosspolymer-3, and in particular Dimethicone Crosspolymer and Dimethicone (and) Dimethicone Crosspolymer.

6. Composition according to any one of the preceding claims, comprising as lipophilic gelling agent at least one gel of silicone elastomer dispersed in a silicone oil and/or a powder of organopolysiloxane elastomer coated with silsesquioxane resin.

**7.** Composition according to any one of the preceding claims, containing, as hydrophilic gelling agent/lipophilic gelling agent system, a 2-acrylamido-2-methylpropanesulfonic acid copolymer/organopolysiloxane elastomer system.

**8.** Composition according to any one of the preceding claims, containing the aqueous and oily phases in an aqueous phase/oily phase weight ratio of from 95/5 to 5/95 and preferably from 30/70 to 80/20.

**9.** Composition according to any one of the preceding claims, in the form of a composition for caring for the skin of the body or the face, in particular the face.

**10.** Composition according to any one of the preceding claims, also comprising at least solid particles such as pigments and/or additional fillers.

**11.** Composition according to any one of the preceding claims, also comprising volatile and/or non-volatile silicone oils.

**12.** Composition according to any one of the preceding claims, also comprising at least one moisturizer, preferably glycerol.

**13.** Process for preparing a cosmetic composition, different from an emulsion, for making up and/or caring for keratin materials, in particular the skin and/or the lips, comprising at least one step of mixing:

- an aqueous phase gelled with at least one synthetic polymeric hydrophilic gelling agent chosen from crosslinked and/or neutralized 2-acrylamido-2-methylpropanesulfonic acid copolymers; and
- at least one oily phase gelled with at least one lipophilic gelling agent chosen from organopolysiloxane elastomers;

under conditions suitable for obtaining a macroscopically homogeneous mixture;
said composition also comprising at least one soft-focus filler chosen from acrylic copolymer powders, polymethyl methacrylate powders, talc/$TiO_2$/alumina/silica composite powders, silica/$TiO_2$ composite powders, hollow hemispherical silicone particles, crosslinked elastomeric organopolysiloxane powders, crosslinked elastomeric organopolysiloxane powders coated with silicone resin, starch powders, polyamide powders, spherical cellulose beads, and mixtures thereof.

**14.** Process according to Claim 13, comprising a step of mixing at least three or even more gelled phases.

**15.** Process according to either of Claims 13 and 14, in which the mixing is performed at room temperature.

**16.** Cosmetic process for making up and/or caring for keratin materials, in particular the skin and/or the lips, comprising at least one step which consists in applying to said keratin material a composition as defined according to any one of Claims 1 to 12.

**17.** Cosmetic process for making up and/or caring for a keratin material, in particular the skin and/or the lips, comprising at least the application to said material of a macroscopically homogeneous composition, different from an emulsion, obtained by extemporaneous mixing, before application or at the time of application to said keratin material, of at least one aqueous phase gelled with at least one synthetic polymeric hydrophilic gelling agent chosen from crosslinked and/or neutralized 2-acrylamido-2-methylpropanesulfonic acid copolymers, and of at least one oily phase gelled with at least one lipophilic gelling agent chosen from organopolysiloxane elastomers; and said composition also comprising at least one soft-focus filler chosen from acrylic copolymer powders, polymethyl methacrylate powders, talc/$TiO_2$/alumina/silica composite powders, silica/$TiO_2$ composite powders, hollow hemispherical silicone particles, crosslinked elastomeric organopolysiloxane powders, crosslinked elastomeric organopolysiloxane powders coated with silicone resin, starch powders, polyamide powders, spherical cellulose beads, and mixtures thereof.

## Patentansprüche

**1.** Kosmetische Zusammensetzung, die von einer Emulsion verschieden ist, zum Schminken und/oder Pflegen von Keratinmaterialien, insbesondere der Haut und/oder der Lippen, die Folgendes umfasst:

- mindestens eine wässerige Phase, die mit mindestens einem synthetischen polymeren hydrophilen Geliermittel geliert ist, das aus vernetzten und/oder neutralisierten 2-Acrylamido-2-Methylpropansulfonsäure-Copolymeren gewählt ist; und
- mindestens eine ölige Phase, die mit mindestens einem lipophilen Geliermittel geliert ist, das aus Organopolysiloxan-Elastomeren gewählt ist;

wobei die Phasen dabei ein makroskopisch homogenes Gemisch bilden;
wobei die Zusammensetzung auch mindestens einen Weichzeichnungsfüllstoff umfasst, der aus Acrylcopolymer-Pulvern, Polymethylmethacrylat-Pulvern, Talkum/TiO2/Aluminiumoxid/Siliziumdioxid-Verbundpulvern, Siliziumdioxid/Ti02-Verbundpulvern, hohlen halbkugelförmigen Silikonpartikeln, vernetzten elastomeren Organopolysiloxan-Pulvern, vernetzten elastomeren Organopolysiloxan-Pulvern, die mit Silikonharz beschichtet sind, Stärkepulvern, Polyamid-Pulvern, kugelförmigen Cellulosekügelchen und Gemischen davon gewählt ist.

**2.** Zusammensetzung nach dem vorhergehenden Anspruch, die 0,2 bis 40 Gew.-%, speziell 0,5 bis 37 Gew.-%, insbesondere 0,75 bis 35 Gew.-% und vorzugsweise 1 bis 30 Gew.-% Weichzeichnungsfüllstoff(e) relativ zum Gesamtgewicht der Zusammensetzung umfasst.

**3.** Zusammensetzung nach einem der vorhergehenden Ansprüche, wobei der (die) Weichzeichnungsfüllstoff(e) insgesamt oder teilweise und vorzugsweise allein in der gelierten wässerigen Phase vorhanden ist (sind) oder insgesamt oder teilweise und vorzugsweise allein in der gelierten öligen Phase vorhanden ist (sind).

**4.** Zusammensetzung nach einem der vorhergehenden Ansprüche, die als synthetisches polymeres hydrophiles Geliermittel mindestens ein Copolymer von 2-Acrylamido-2-methylpropansulfonsäure und von Hydroxyethylacrylat umfasst.

**5.** Zusammensetzung nach einem der vorhergehenden Ansprüche, wobei das lipophile Geliermittel aus Dimethicon-Kreuzpolymer, Dimethicon (und) Dimethicon-Kreuzpolymer, Vinyldimethicon-Kreuzpolymer, Dimethicon/Vinyldimethicon-Kreuzpolymer, Dimethicon-Kreuzpolymer-3 und insbesondere Dimethicon-Kreuzpolymer und Dimethicon (und) Dimethicon-Kreuzpolymer gewählt ist.

**6.** Zusammensetzung nach einem der vorhergehenden Ansprüche, die als lipophiles Geliermittel mindestens ein Gel aus Silikonelastomer, das in einem Silikonöl dispergiert ist, und/oder ein Pulver von Organopolysiloxan-Elastomer, das mit Silesquioxan-Harz beschichtet ist, umfasst.

**7.** Zusammensetzung nach einem der vorhergehenden Ansprüche, die als System aus hydrophilem Geliermittel/lipophilem Geliermittel ein System aus 2-Acrylamido-2-methylpropansulfonsäure-Copolymer/Organopolysiloxan-Elastomer enthält.

**8.** Zusammensetzung nach einem der vorhergehenden Ansprüche, die die wässerige und die ölige Phase in einem Gewichtsverhältnis der wässerigen Phase/öligen Phase von 95/5 bis 5/95 und vorzugsweise von 30/70 bis 80/20 enthält.

**9.** Zusammensetzung nach einem der vorhergehenden Ansprüche in Form einer Zusammensetzung zum Pflegen der Haut des Körpers oder des Gesichts, insbesondere des Gesichts.

**10.** Zusammensetzung nach einem der vorhergehenden Ansprüche, die auch zumindest feste Partikel wie z. B. Pigmente und/oder zusätzliche Füllstoffe umfasst.

**11.** Zusammensetzung nach einem der vorhergehenden Ansprüche, die auch flüchtige und/oder nichtflüchtige Silikonöle umfasst.

**12.** Zusammensetzung nach einem der vorhergehenden Ansprüche, die auch mindestens ein Befeuchtungsmittel, vorzugsweise Glycerin, umfasst.

**13.** Verfahren zur Herstellung einer kosmetischen Zusammensetzung, die von einer Emulsion verschieden ist, zum Schminken und/oder Pflegen von Keratinmaterialien, insbesondere der Haut und/oder der Lippen, mit mindestens einem Schritt zum Mischen:

- einer wässerigen Phase, die mit mindestens einem synthetischen polymeren hydrophilen Geliermittel geliert ist, das aus vernetzten und/oder neutralisierten 2-Acrylamido-2-Methylpropansulfonsäure-Copolymeren gewählt ist; und
- mindestens einer öligen Phase, die mit mindestens einem lipophilen Geliermittel geliert ist, das aus Organopolysiloxan-Elastomeren gewählt ist;

unter Bedingungen, die für das Erhalten eines makroskopisch homogenen Gemisches geeignet sind; wobei die Zusammensetzung auch mindestens einen Weichzeichnungsfüllstoff umfasst, der aus Acrylcopolymer-Pulvern, Polymethylmethacrylat-Pulvern, Talkum/TiO2/Aluminiumoxid/Siliziumdioxid-Verbundpulvern, Siliziumdioxid/Ti02-Verbundpulvern, hohlen halbkugelförmigen Silikonpartikeln, vernetzten elastomeren Organopolysiloxan-Pulvern, vernetzten elastomeren Organopolysiloxan-Pulvern, die mit Silikonharz beschichtet sind, Stärkepulvern, Polyamid-Pulvern, kugelförmigen Cellulosekügelchen und Gemischen davon gewählt ist.

**14.** Verfahren nach Anspruch 13, das einen Schritt zum Mischen von mindestens drei oder noch mehr gelierten Phasen umfasst.

**15.** Verfahren nach einem der Ansprüche 13 und 14, wobei das Mischen bei Raumtemperatur durchgeführt wird.

**16.** Kosmetisches Verfahren zum Schminken und/oder Pflegen von Keratinmaterialien, insbesondere der Haut und/oder der Lippen, mit mindestens einem Schritt, der aus dem Aufbringen einer Zusammensetzung, wie gemäß irgendeinem der Ansprüche 1 bis 12 definiert, auf das Keratinmaterial umfasst.

**17.** Kosmetisches Verfahren zum Schminken und/oder Pflegen eines Keratinmaterials, insbesondere der Haut und/oder der Lippen, mit zumindest dem Aufbringen einer makroskopisch homogenen Zusammensetzung, die von einer Emulsion verschieden ist, auf das Material, die erhalten wird durch unvorbereitetes Mischen, vor dem Aufbringen oder zur Zeit des Aufbringens auf das Keratinmaterial, von mindestens einer wässerigen Phase, die mit mindestens einem synthetischen polymeren hydrophilen Geliermittel geliert ist, das aus vernetzten und/oder neutralisierten 2-Acrylamido-2-methylpropansulfonsäure-Copolymeren gewählt ist, und mindestens einer öligen Phase, die mit mindestens einem lipophilen Geliermittel geliert ist, das aus Organopolysiloxan-Elastomeren gewählt ist; und wobei die Zusammensetzung auch mindestens einen Weichzeichnungsfüllstoff umfasst, der aus Acrylcopolymer-Pulvern, Polymethylmethacrylat-Pulvern, Talkum/TiO2/Aluminiumoxid/Siliziumdioxid-Verbundpulvern, Siliziumdioxid/$TiO_2$-Verbundpulvern, hohlen halbkugelförmigen Silikonpartikeln, vernetzten elastomeren Organopolysiloxan-Pulvern, vernetzten elastomeren Organopolysiloxan-Pulvern, die mit Silikonharz beschichtet sind, Stärkepulvern, Polyamid-Pulvern, kugelförmigen Cellulosekügelchen und Gemischen davon gewählt ist.

## Revendications

**1.** Composition cosmétique, différente d'une émulsion, pour le maquillage et/ou le soin des matières kératiniques, en particulier la peau et/ou les lèvres, comprenant :

- au moins une phase aqueuse gélifiée avec au moins un agent gélifiant hydrophile polymérique synthétique choisi parmi les copolymères d'acide 2-acrylamido-2-méthylpropanesulfonique réticulés et/ou neutralisés ; et
- au moins une phase huileuse gélifiée avec au moins un agent gélifiant lipophile choisi parmi les élastomères d'organopolysiloxane ;

lesdites phases y formant un mélange macroscopiquement homogène ;
ladite composition comprenant aussi au moins une charge à effet flouteur choisie parmi les poudres de copolymère acrylique, les poudres de poly(méthacrylate de méthyle), les poudres composites de talc/$TiO_2$/alumine/silice, les poudres composites de silice/$TiO_2$, les particules siliconées hémisphériques creuses, les poudres d'organopolysiloxane élastomère réticulé, les poudres d'organopolysiloxane élastomère réticulé revêtues de résine siliconée, les poudres d'amidon, les poudres de polyamide, les perles de cellulose sphériques, et leurs mélanges.

**2.** Composition selon la revendication précédente, comprenant de 0,2 % à 40 % en poids, tout spécialement de 0,5 % à 37 % en poids, en particulier de 0,75 % à 35 % en poids et de préférence de 1 % à 30 % en poids d'une ou plusieurs charges à effet flouteur par rapport au poids total de ladite composition.

3. Composition selon l'une ou l'autre des revendications précédentes, dans laquelle ladite ou lesdites charges à effet flouteur sont totalement ou partiellement, et de préférence uniquement, présentes dans la phase aqueuse gélifiée ou sont totalement ou partiellement, et de préférence uniquement, présentes dans la phase huileuse gélifiée.

4. Composition selon l'une quelconque des revendications précédentes, comprenant, en tant qu'agent gélifiant hydrophile polymérique synthétique, au moins un copolymère d'acide 2-acrylamido-2-méthylpropanesulfonique et d'acrylate d'hydroxyéthyle.

5. Composition selon l'une quelconque des revendications précédentes, dans laquelle l'agent gélifiant lipophile est choisi parmi un polymère réticulé de diméthicone, un polymère réticulé de diméthicone (et) de diméthicone, un polymère réticulé de vinyldiméthicone, un polymère réticulé de diméthicone/vinyldiméthicone, un polymère réticulé de diméthicone-3, et en particulier un polymère réticulé de diméthicone et un polymère réticulé de diméthicone (et) de diméthicone.

6. Composition selon l'une quelconque des revendications précédentes, comprenant, en tant qu'agent gélifiant lipophile, au moins un gel d'élastomère siliconé dispersé dans une huile siliconée et/ou une poudre d'élastomère d'organopolysiloxane revêtue de résine de silsesquioxane.

7. Composition selon l'une quelconque des revendications précédentes, contenant, en tant que système d'agent gélifiant hydrophile/agent gélifiant lipophile, un système de copolymère d'acide 2-acrylamido-2-méthylpropanesulfonique/élastomère d'organopolysiloxane.

8. Composition selon l'une quelconque des revendications précédentes, contenant les phases aqueuse et huileuse en un rapport en poids phase aqueuse/phase huileuse de 95/5 à 5/95 et de préférence de 30/70 à 80/20.

9. Composition selon l'une quelconque des revendications précédentes, sous la forme d'une composition pour le soin de la peau du corps ou du visage, en particulier du visage.

10. Composition selon l'une quelconque des revendications précédentes, comprenant aussi au moins des particules solides telles que des pigments et/ou des charges additionnelles.

11. Composition selon l'une quelconque des revendications précédentes, comprenant aussi des huiles siliconées volatiles et/ou non volatiles.

12. Composition selon l'une quelconque des revendications précédentes, comprenant aussi au moins un agent hydratant, de préférence le glycérol.

13. Procédé pour préparer une composition cosmétique, différente d'une émulsion, pour le maquillage et/ou le soin des matières kératiniques, en particulier la peau et/ou les lèvres, comprenant au moins une étape de mélange :

- d'une phase aqueuse gélifiée avec au moins un agent gélifiant hydrophile polymérique synthétique choisi parmi les copolymères d'acide 2-acrylamido-2-méthylpropanesulfonique réticulés et/ou neutralisés ; et
- d'au moins une phase huileuse gélifiée avec au moins un agent gélifiant lipophile choisi parmi les élastomères d'organopolysiloxane ;

dans des conditions convenant à l'obtention d'un mélange macroscopiquement homogène ;
ladite composition comprenant aussi au moins une charge à effet flouteur choisie parmi les poudres de copolymère acrylique, les poudres de poly(méthacrylate de méthyle), les poudres composites de talc/$TiO_2$/alumine/silice, les poudres composites de silice/$TiO_2$, les particules siliconées hémisphériques creuses, les poudres d'organopolysiloxane élastomère réticulé, les poudres d'organopolysiloxane élastomère réticulé revêtues de résine siliconée, les poudres d'amidon, les poudres de polyamide, les perles de cellulose sphériques, et leurs mélanges.

14. Procédé selon la revendication 13, comprenant une étape de mélange d'au moins trois ou même plus de trois phases gélifiées.

15. Procédé selon l'une ou l'autre des revendications 13 et 14, dans lequel le mélange est effectué à la température ambiante.

**16.** Procédé cosmétique de maquillage et/ou de soin des matières kératiniques, en particulier de la peau et/ou des lèvres, comprenant au moins une étape qui consiste à appliquer à ladite matière kératinique une composition telle que définie selon l'une quelconque des revendications 1 à 12.

**17.** Procédé cosmétique de maquillage et/ou de soin des matières kératiniques, en particulier de la peau et/ou des lèvres, comprenant au moins l'application sur ladite matière d'une composition macroscopiquement homogène, différente d'une émulsion, obtenue par mélange extemporané, avant l'application ou au moment de l'application sur ladite matière kératinique, d'au moins une phase aqueuse gélifiée avec au moins un agent gélifiant hydrophile polymérique synthétique choisi parmi les copolymères d'acide 2-acrylamido-2-méthylpropanesulfonique réticulés et/ou neutralisés, et d'au moins une phase huileuse gélifiée avec au moins un agent gélifiant lipophile choisi parmi les élastomères d'organopolysiloxane ; et ladite composition comprenant aussi au moins une charge à effet flouteur choisie parmi les poudres de copolymère acrylique, les poudres de poly(méthacrylate de méthyle), les poudres composites de talc/$TiO_2$/alumine/silice, les poudres composites de silice/$TiO_2$, les particules siliconées hémisphériques creuses, les poudres d'organopolysiloxane élastomère réticulé, les poudres d'organopolysiloxane élastomère réticulé revêtues de résine siliconée, les poudres d'amidon, les poudres de polyamide, les perles de cellulose sphériques, et leurs mélanges.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9965455 A
- WO 04057589 A
- WO 9962497 A
- JP 2005112834 A
- WO 2013093869 A
- WO 2008081175 A
- WO 9922696 A
- EP 0216479 A
- US 3915921 A
- US 4509949 A
- WO 9844012 A
- EP 0815928 B1
- US 7470725 B
- EP 295886 A
- EP 242219 A
- EP 285886 A
- EP 765656 A
- JP 61194009 A
- US 5538793 A
- EP 0951897 A
- US 5156911 A
- WO 0119333 A
- US 5981680 A
- JP 2003128788 B
- JP 2000191789 A
- WO 2008155059 A
- JP 09188830 A
- JP 10158450 A
- JP 10158541 A
- JP 07258460 A
- JP 05017710 A
- EP 1086683 A


### Non-patent literature cited in the description


- **ALMEIDA et al.** *Pharmaceutical Development and Technology,* 2008, vol. 13 (487), 488
- **REMINGTON.** Remington: The Science and Practice of Pharmacy. 1995, 282
- **G. FONNUM ; J. BAKKE ; FK. HANSEN.** *Colloid Polym. Sci.,* 1993, vol. 271, 380-389
- **C.M. HANSEN.** The three-dimensional solubility parameters. *J. Paint Technol,* 1967, vol. 39, 105
- **BRINKER C.J ; SCHERER G.W.** Sol-Gel Science. Academic Press, 1990
- *Journal of the American Chemical Society,* February 1938, vol. 60, 309
- **VAN DE HULST, H.C.** Light Scattering by Small Particles. Wiley, 1957